(19) Europäisches Patentamt / European Patent Office / Office européen des brevets

(11) **EP 4 393 506 A1**

(12) **EUROPEAN PATENT APPLICATION**
published in accordance with Art. 153(4) EPC

(43) Date of publication:
**03.07.2024 Bulletin 2024/27**

(21) Application number: **22860563.0**

(22) Date of filing: **25.08.2022**

(51) International Patent Classification (IPC):
**A61K 39/395** (2006.01)   **C07K 16/24** (2006.01)
**A61K 38/17** (2006.01)   **C07K 16/28** (2006.01)
**A61P 35/00** (2006.01)   **A61P 37/00** (2006.01)

(52) Cooperative Patent Classification (CPC):
**A61K 38/17; A61K 39/395; A61P 35/00;**
**A61P 37/00; C07K 16/24; C07K 16/28**

(86) International application number:
**PCT/CN2022/114652**

(87) International publication number:
**WO 2023/025217 (02.03.2023 Gazette 2023/09)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **26.08.2021   CN 202110988008**

(71) Applicant: **CHIA TAI TIANQING**
**PHARMACEUTICAL GROUP CO., LTD.**
**Lianyungang,**
**Jiangsu 222062 (CN)**

(72) Inventors:
• **CHENG, Yanju**
**Nanjing, Jiangsu 211100 (CN)**

• **LI, Yingchun**
**Nanjing, Jiangsu 211100 (CN)**
• **KONG, Lingjie**
**Nanjing, Jiangsu 211100 (CN)**
• **GUO, Xiaolu**
**Nanjing, Jiangsu 211100 (CN)**
• **ZHAO, Wei**
**Nanjing, Jiangsu 211100 (CN)**

(74) Representative: **Grünecker Patent- und**
**Rechtsanwälte**
**PartG mbB**
**Leopoldstraße 4**
**80802 München (DE)**

Remarks:
The complete document including Reference
Table(s) and the Sequence Listing(s) can be
downloaded from the EPO website

(54) **PHARMACEUTICAL COMPOSITION OF ANTI-IL4R ANTIBODY AND USE THEREOF**

(57)     The present disclosure provides a pharmaceutical composition of an anti-IL4R antibody and a use thereof. The pharmaceutical composition comprises the anti-IL4R antibody or an antigen binding fragment thereof, and a buffering agent; and the pharmaceutical composition can further comprise a surfactant and a stabilizer.

EP 4 393 506 A1

**Description**

**TECHNICAL FIELD**

The present disclosure relates to the field of formulation, and in particular, to a stable pharmaceutical composition comprising an anti-IL4R antibody or an antigen-binding fragment thereof, and use of the pharmaceutical composition in treating and/or preventing a disease associated with excessive IL4 and/or IL13 signaling.

**BACKGROUND**

Type 2 inflammation-related allergic diseases, such as atopic dermatitis, anaphylaxis, allergic rhinitis, and allergic asthma, afflict more than 3 billion people worldwide, and exhibit a continuously increasing incidence. According to the hygiene hypothesis, the high incidence is partly attributed to a lack of exposure to infectious agents due to the increasing living standard, which makes the immune system more sensitive to certain otherwise harmless allergens (Stephen J. Galli et al., (2008) Nature 454(7203):445-454). Interleukin 4 (IL4 or IL-4) and IL-13 are two key factors in type 2 immunity and are required to drive the majority of key markers associated with type 2 inflammation, e.g., immunoglobulin E production and recruitment of innate cells at the lesions of inflammation (Gruning G et al., (1998) Science 282:2261-2263; Rankin JA et al., (1996) Proc Natl Acad Sci USA 93:7821-7825; Wills-Karp M et al., (1998) Science 282:2258-2261).

IL-4 and IL-13 regulate cell functionality and activate transcription machinery by binding to cell surface receptors. Specifically, IL-4 first binds to the IL-4R$\alpha$ chain with a picomolar affinity, and then recruits IL-2R$\gamma$ ($\gamma$c) to form a type I receptor complex or recruits IL-13R$\alpha$1 to form a type II receptor complex. It was found that nonhematopoietic cells demonstrate no expression or underexpression of $\gamma$c but high expression of IL-13R$\alpha$1, whereas lymphocytes do not. Bone marrow cells are intermediate of the two types of cells. Type II receptor complexes can also be formed by the binding of IL-13 to the IL-13R$\alpha$1 chain (with a nanomolar affinity) and further recruiting the IL-4R$\alpha$ chain. In addition, IL-13 is also capable of binding to IL-13R$\alpha$2 with a picomolar affinity to initiate the decoy receptor formation (Irina G. Luzina et al., (2012) J Leukoc Biol 92(4):753-764). Once the IL-4 receptor complex is formed, intracellular signaling molecules are activated, with STAT6 and IRS signaling pathways activated in response to the type I IL-4 receptor, whereas the type II IL-4 receptor does not significantly activate IRS (Heller NM et al., (2008) Sci Signal 1(51):ra17-ra17). STAT6 signaling is important for $T_H2$ cell differentiation and IL-4 production, while IRS can activate both PI3K and mTOR signaling pathways (Gadani SP et al., (2012) J Immunol 189:4213-4219). Studies have shown that excessive IL-4 and/or IL-13 signaling may cause allergic diseases. STAT6 inhibitors have also been found to inhibit the growth of prostate cancer cells (Nappo G et al., (2017) Oncogenesis 6(5):e342). Thus, several therapeutic antibodies that modulate IL-4- and/or IL-13-mediated signaling were developed, e.g., the antibody dupilumab against IL4R.

Antibody drugs for human subjects should retain their biological activity and stability during the preservation and subsequent use, but the antibody drugs have large molecular weights and complex structures, and are susceptible to degradation, aggregation, undesirable chemical modifications, etc., so as to become unstable. Accordingly, there is a need in the art for a stable antibody pharmaceutical composition (e.g., an anti-IL4R antibody pharmaceutical composition) that overcomes the above-mentioned defects, possesses a longer shelf life, and is suitable for manufacture and administration to patients. However, when antibodies are present at a high concentration, it is still required to solve problems such as high aggregation, osmotic pressures higher than the physiological level, and in particular, low injectability due to increased viscosity.

**BRIEF SUMMARY**

The present disclosure provides a pharmaceutical composition comprising an anti-IL4R$\alpha$ antibody or an antigen-binding fragment thereof at a high concentration, having an acceptable viscosity. In addition, the pharmaceutical composition of the present disclosure does not result in a high level of aggregation.

The present disclosure provides a pharmaceutical composition, comprising: (a) an anti-IL4R$\alpha$ antibody or an antigen-binding fragment thereof, (b) a buffering agent, (c) a surfactant, and (d) a stabilizer.

In some embodiments, the concentration of the anti-IL4R$\alpha$ antibody or the antigen-binding fragment thereof in the aforementioned pharmaceutical composition is 30 mg/mL to 300 mg/mL, 50 mg/mL to 250 mg/mL, 70 mg/mL to 200 mg/mL, 100 mg/mL to 180 mg/mL, 120 mg/mL to 180 mg/mL, 120 mg/mL to 150 mg/mL, or 150 mg/mL to 180 mg/mL. In some embodiments, the concentration of the anti-IL4R$\alpha$ antibody or the antigen-binding fragment thereof in the aforementioned pharmaceutical composition is about 150 mg/mL. In some embodiments, the concentration of the anti-IL4R$\alpha$ antibody or the antigen-binding fragment thereof in the aforementioned pharmaceutical composition is about 175 mg/mL. In some embodiments, examples of the concentration of the anti-IL4R$\alpha$ antibody or the antigen-binding fragment thereof in the aforementioned pharmaceutical composition include, but are not limited to: about 70 mg/mL, about 80 mg/mL, about 90 mg/mL, about 100 mg/mL, about 110 mg/mL, about 120 mg/mL, about 125 mg/mL, about 130 mg/mL, about 135 mg/mL, about 140 mg/mL, about 145 mg/mL, about 150 mg/mL, about 155 mg/mL, about 160 mg/mL, about 165 mg/mL, about 170 mg/mL, about 175 mg/mL, about 180 mg/mL, about 190 mg/mL, or about 200 mg/mL.

In some embodiments, examples of the buffering agent in the aforementioned pharmaceutical composition include a phosphate buffering agent, an acetate buffering agent, or a histidine salt buffering agent. Preferably, examples of the buffering agent in the aforementioned pharmaceutical composition include a histidine salt buffering agent. In some specific embodiments, examples of the phosphate buffering agent include a sodium phosphate buffering agent or a potassium phosphate buffering agent, examples of the acetate buffering agent include a sodium acetate buffering agent, a potassium acetate buffering agent, or a sodium acetate-acetic acid buffering agent, and examples of the histidine salt buffering agent include a histidine-hydrochloric acid buffering agent, a histidine-acetic acid buffering agent, a histidine-histidine hydrochloride buffering agent, or a histidine hydrochloride buffering agent. In some specific embodiments, the sodium phosphate buffering agent comprises disodium hydrogen phosphate and sodium dihydrogen phosphate. In some specific embodiments, the sodium acetate-acetic acid buffering agent comprises sodium acetate and acetic acid. In some specific embodiments, the histidine-histidine hydrochloride buffering agent comprises histidine and histidine hydrochloride.

In some embodiments, the concentration of the buffering agent in the aforementioned pharmaceutical composition is 1 mM to 100 mM, 2 mM to 80 mM, 4 mM to 60 mM, 8 mM to 40 mM, 10 mM to 30 mM, 10 mM to 20 mM, or 20 mM to 30 mM. In some embodiments, the concentration of the buffering agent in the aforementioned pharmaceutical composition is about 20 mM. In some embodiments, examples of the concentration of the buffering agent in the aforementioned pharmaceutical composition include, but are not limited to: about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 26 mM, about 27 mM, about 28 mM, about 30 mM, about 32 mM, about 34 mM, about 36 mM, about 38 mM, or about 40 mM.

In some embodiments, the pH of the buffering agent in the aforementioned pharmaceutical composition is 4 to 7, 5 to 7, 5 to 6.5, 5.3 to 6.5, 5.3 to 6.3, 5.3 to 5.8, or 5.8 to 6.3. In some embodiments, the pH of the buffering agent in the aforementioned pharmaceutical composition is about 5.8. In some embodiments, the pH of the buffering agent in the aforementioned pharmaceutical composition is about 6. In some embodiments, the pH of the buffering agent in the aforementioned pharmaceutical composition is about 5. In some embodiments, examples of the pH of the buffering agent in the aforementioned pharmaceutical composition include, but are not limited to: about 5, about 5.1, about 5.2, about 5.3, about 5.4, about 5.5, about 5.6, about 5.7, about 5.8, about 5.9, about 6, about 6.1, about 6.2, about 6.3, about 6.4, about 6.5, about 6.6, about 6.7, about 6.8, about 6.9, or about 7.

In some embodiments, the surfactant in the aforementioned pharmaceutical composition is selected from a polysorbate (e.g., polysorbate 20, polysorbate 40, polysorbate 60, polysorbate 65, polysorbate 80, polysorbate 81, or polysorbate 85), a poloxamer (e.g., poloxamer 181, poloxamer 188, or poloxamer 407), polyethylene glycol, a polyhydroxy compound, and the like. In some embodiments, the surfactant in the aforementioned pharmaceutical composition is polysorbate 80. In some embodiments, the surfactant in the aforementioned pharmaceutical composition is polysorbate 20.

In some embodiments, the concentration of the surfactant in the aforementioned pharmaceutical composition is 0.01 mg/mL to 2 mg/mL, 0.05 mg/mL to 1 mg/mL, 0.1 mg/mL to 0.8 mg/mL, 0.2 mg/mL to 0.6 mg/mL, or 0.2 mg/mL to 0.4 mg/mL. In some embodiments, the concentration of the surfactant in the aforementioned pharmaceutical composition is about 0.4 mg/mL. In some embodiments, examples of the concentration of the surfactant in the aforementioned pharmaceutical composition include, but are not limited to: about 0.05 mg/mL, about 0.06 mg/mL, about 0.07 mg/mL, about 0.08 mg/mL, about 0.09 mg/mL, about 0.1 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, or about 1 mg/mL.

In some embodiments, the stabilizer in the aforementioned pharmaceutical composition is selected from one or more of trehalose, mannitol, sucrose, arginine or a pharmaceutically acceptable salt thereof, proline, and sodium chloride. In some embodiments, the stabilizer in the aforementioned pharmaceutical composition comprises sucrose. In some embodiments, the stabilizer in the aforementioned pharmaceutical composition comprises arginine or the pharmaceutically acceptable salt thereof. In some embodiments, the stabilizer in the aforementioned pharmaceutical composition comprises proline. In some embodiments, the stabilizer in the aforementioned pharmaceutical composition comprises sodium chloride. Examples of the pharmaceutically acceptable salt of arginine include arginine hydrochloride or arginine acetate. In some specific embodiments, the stabilizer in the aforementioned pharmaceutical composition comprises sucrose and arginine hydrochloride. In some specific embodiments, the stabilizer in the aforementioned pharmaceutical composition comprises proline and sodium chloride. In some specific embodiments, the stabilizer in the aforementioned pharmaceutical composition comprises sucrose, proline, and sodium chloride. In some embodiments, the concentration of sucrose in the aforementioned pharmaceutical composition is 10 mg/mL to 100 mg/mL, 20 mg/mL to 80 mg/mL, 30 mg/mL to 60 mg/mL, 40 mg/mL to 50 mg/mL, or 50 mg/mL to 60 mg/mL. In some embodiments, the concentration of sucrose in the aforementioned pharmaceutical composition is about 50 mg/mL. In some embodiments, examples of the concentration of sucrose in the aforementioned pharmaceutical composition include, but are not limited to: about 20 mg/mL, about 22 mg/mL, about 24 mg/mL, about 26 mg/mL, about 28 mg/mL, about 30 mg/mL, about 32 mg/mL, about 34 mg/mL, about 36 mg/mL, about 38 mg/mL, about 40 mg/mL, about 42 mg/mL, about 44 mg/mL, about 46 mg/mL, about 48 mg/mL, about 50 mg/mL, about 52 mg/mL, about 54 mg/mL, about 56 mg/mL, about 58 mg/mL, about 60 mg/mL, about 62 mg/mL, about 64 mg/mL, about 66 mg/mL, about 68 mg/mL, about 70 mg/mL, about 72 mg/mL, about 74 mg/mL, about 76 mg/mL, about 78 mg/mL, or about 80 mg/mL.

In some embodiments, the concentration of arginine or the pharmaceutically acceptable salt thereof (e.g., arginine hydrochloride) in the aforementioned pharmaceutical composition is 10 mM to 100 mM, 20 mM to 80 mM, 30 mM to 60 mM, 40 mM to 50 mM, or 50 mM to 60 mM. In some embodiments, the concentration of arginine or the pharmaceutically acceptable salt thereof (e.g., arginine hydrochloride) in the aforementioned pharmaceutical composition is about 50 mM. In some embodiments, examples of the concentration of arginine or the pharmaceutically acceptable salt thereof (e.g., arginine hydrochloride) in the aforementioned pharmaceutical composition include, but are not limited to: about 20 mM, about 22 mM, about 24 mM, about 26 mM, about 28 mM, about 30 mM, about 32 mM, about 34 mM, about 36 mM, about 38 mM, about 40 mM, about 42 mM, about 44 mM, about 46 mM, about 48 mM, about 50 mM, about 52 mM, about 54 mM, about 56 mM, about 58 mM, about 60 mM, about 62 mM, about 64 mM, about 66 mM, about 68 mM, about 70 mM, about 72 mM, about 74 mM, about 76 mM, about 78 mM, or about 80 mM.

In some embodiments, the concentration of proline in the aforementioned pharmaceutical composition is 60 mM to 600 mM, 80 mM to 500 mM, 100 mM to 450 mM, 120 mM to 400 mM, 150 mM to 350 mM, 200 mM to 350 mM, or 250 mM to 350 mM. In some embodiments, the concentration of proline in the aforementioned pharmaceutical composition is about 250 mM. In some embodiments, the concentration of proline in the aforementioned pharmaceutical composition is about 350 mM. In some embodiments, examples of the concentration of proline in the aforementioned pharmaceutical composition include, but are not limited to: about 120 mM, about 130 mM, about 140 mM, about 150 mM, about 160 mM, about 170 mM, about 180 mM, about 190 mM, about 200 mM, about 210 mM, about 220 mM, about 230 mM, about 240 mM, about 250 mM, about 260 mM, about 270 mM, about 280 mM, about 290 mM, about 300 mM, about 310 mM, about 320 mM, about 330 mM, about 340 mM, about 350 mM, about 360 mM, about 370 mM, about 380 mM, about 390 mM, or about 400 mM.

In some embodiments, the concentration of sodium chloride in the aforementioned pharmaceutical composition is 1 mM to 80 mM, 2 mM to 60 mM, 4 mM to 50 mM, 8 mM to 40 mM, 10 mM to 30 mM, or 10 mM to 20 mM. In some embodiments, the concentration of sodium chloride in the aforementioned pharmaceutical composition is about 20 mM. In some embodiments, examples of the concentration of sodium chloride in the aforementioned pharmaceutical composition include, but are not limited to: about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 26 mM, about 27 mM, about 28 mM, about 29 mM, about 30 mM, about 32 mM, about 34 mM, about 36 mM, about 38 mM, or about 40 mM.

In some embodiments, the pH of the aforementioned pharmaceutical composition is 4 to 7, 5 to 7, 5 to 6.5, 5.3 to 6.5, 5.3 to 6.3, 5.3 to 5.8, or 5.8 to 6.3. In some embodiments, the pH of the aforementioned pharmaceutical composition is about 5.8. In some embodiments, the pH of the aforementioned pharmaceutical composition is about 6. In some embodiments, the pH of the aforementioned pharmaceutical composition is about 5. In some embodiments, examples of the pH of the aforementioned pharmaceutical composition include, but are not limited to: about 5, about 5.1, about 5.2, about 5.3, about 5.4, about 5.5, about 5.6, about 5.7, about 5.8, about 5.9, about 6, about 6.1, about 6.2, about 6.3, about 6.4, about 6.5, about 6.6, about 6.7, about 6.8, about 6.9, or about 7.

In some embodiments, the aforementioned pharmaceutical composition comprises: (a) the anti-IL4Rα antibody or the antigen-binding fragment thereof; (b) the phosphate buffering agent, the acetate buffering agent, and/or the histidine salt buffering agent; (c) polysorbate 80 or polysorbate 20; and (d) trehalose, mannitol, sucrose, arginine or the pharmaceutically acceptable salt thereof, proline, and/or sodium chloride.

In some embodiments, the aforementioned pharmaceutical composition comprises: (a) the anti-IL4Ra antibody or the antigen-binding fragment thereof; (b) the phosphate buffering agent or the histidine salt buffering agent; (c) polysorbate 80; and (d) sucrose, arginine or the pharmaceutically acceptable salt thereof, proline, and/or sodium chloride.

In some embodiments, the aforementioned pharmaceutical composition comprises: (a) the anti-IL4Rα antibody or the antigen-binding fragment thereof; (b) the histidine salt buffering agent; (c) polysorbate 80; and (d) sucrose and arginine or the pharmaceutically acceptable salt thereof.

In some embodiments, the aforementioned pharmaceutical composition comprises: (a) the anti-IL4Rα antibody or the antigen-binding fragment thereof; (b) the histidine salt buffering agent; (c) polysorbate 80; and (d) sucrose, proline, and sodium chloride.

In some embodiments, the aforementioned pharmaceutical composition comprises: (a) the anti-IL4Rα antibody or the antigen-binding fragment thereof; (b) the histidine salt buffering agent; (c) polysorbate 80; and (d) proline and sodium chloride.

In some embodiments, the aforementioned pharmaceutical composition comprises: (a) the anti-IL4Rα antibody or the antigen-binding fragment thereof; (b) the histidine salt buffering agent; (c) polysorbate 80; and (d) proline.

In some embodiments, the aforementioned pharmaceutical composition comprises: (a) 30 mg/mL to 300 mg/mL of the anti-IL4Rα antibody or the antigen-binding fragment thereof; (b) 1 mM to 100 mM of the buffering agent; (c) 0.01 mg/mL to 2 mg/mL of the surfactant; and (d) the stabilizer, including trehalose, mannitol, sucrose, arginine or the pharmaceutically acceptable salt thereof, proline, and/or sodium chloride, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 50 mg/mL to 250 mg/mL of the anti-IL4Rα antibody or the antigen-binding fragment thereof; (b) 2 mM to 80 mM of the buffering agent; (c) 0.05 mg/mL to 1 mg/mL of the surfactant; and (d) the stabilizer, including trehalose, mannitol, sucrose, arginine or the pharmaceutically acceptable salt thereof, proline, and/or sodium chloride, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 70 mg/mL to 200 mg/mL of the anti-IL4Rα antibody or the antigen-binding fragment thereof; (b) 4 mM to 60 mM of the buffering agent; (c) 0.1 mg/mL to 0.8 mg/mL of the surfactant; and (d) the stabilizer, including trehalose, mannitol, sucrose, arginine or the pharmaceutically acceptable salt thereof, proline, and/or sodium chloride, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 100 mg/mL to 180 mg/mL or 120 mg/mL to 180 mg/mL of the anti-IL4Rα antibody or the antigen-binding fragment thereof; (b) 8 mM to 40 mM or 10 mM to 30 mM of the buffering agent; (c) 0.2 mg/mL to 0.6 mg/mL of the surfactant; and (d) the stabilizer, including trehalose, mannitol, sucrose, arginine or the pharmaceutically acceptable salt thereof, proline, and/or sodium chloride, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 120 mg/mL to 150 mg/mL or 150 mg/mL to 180 mg/mL of the anti-IL4Rα antibody or the antigen-binding fragment thereof; (b) 10 mM to 20 mM or 20 mM to 30 mM of the buffering agent; (c) 0.2 mg/mL to 0.4 mg/mL of the surfactant; and (d) the stabilizer, including trehalose, mannitol, sucrose, arginine or the pharmaceutically acceptable salt thereof, proline, and/or sodium chloride, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 30 mg/mL to 300 mg/mL of the anti-IL4Rα antibody or the antigen-binding fragment thereof; (b) 1 mM to 100 mM of the phosphate buffering agent, the acetate buffering agent, or the histidine salt buffering agent; (c) 0.01 mg/mL to 2 mg/mL of polysorbate 80 or polysorbate 20; and (d) 10 mg/mL to 100 mg/mL of sucrose, 10 mM to 100 mM of arginine or the pharmaceutically acceptable salt thereof, 60 mM to 600 mM of proline, and/or 1 mM to 80 mM of sodium chloride, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 50 mg/mL to 250 mg/mL of the anti-IL4Rα antibody or the antigen-binding fragment thereof; (b) 2 mM to 80 mM of the phosphate buffering agent, the acetate buffering agent, or the histidine salt buffering agent; (c) 0.05 mg/mL to 1 mg/mL of polysorbate 80 or polysorbate 20; and (d) 20 mg/mL to 80 mg/mL of sucrose, 20 mM to 80 mM of arginine or the pharmaceutically acceptable salt thereof, 80 mM to 500 mM of proline, and/or 2 mM to 60 mM of sodium chloride, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 70 mg/mL to 200 mg/mL of the anti-IL4Rα antibody or the antigen-binding fragment thereof; (b) 4 mM to 60 mM of the phosphate buffering agent, the acetate buffering agent, or the histidine salt buffering agent; (c) 0.1 mg/mL to 0.8 mg/mL of polysorbate 80 or polysorbate 20; and (d) 30 mg/mL to 60 mg/mL of sucrose, 30 mM to 60 mM of arginine or the pharmaceutically acceptable salt thereof, 100 mM to 450 mM or 120 mM to 400 mM of proline, and/or 4 mM to 50 mM of sodium chloride, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 100 mg/mL to 180 mg/mL or 120 mg/mL to 180 mg/mL of the anti-IL4Rα antibody or the antigen-binding fragment thereof; (b) 8 mM to 40 mM or 10 mM to 30 mM of the phosphate buffering agent, the acetate buffering agent, or the histidine salt buffering agent; (c) 0.2 mg/mL to 0.6 mg/mL of polysorbate 80 or polysorbate 20; and (d) 40 mg/mL to 50 mg/mL of sucrose, 40 mM to 50 mM of arginine or the pharmaceutically acceptable salt thereof, 150 mM to 350 mM of proline, and/or 8 mM to 40 mM of sodium chloride, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 120 mg/mL to 150 mg/mL or 150 mg/mL to 180 mg/mL of the anti-IL4Rα antibody or the antigen-binding fragment thereof; (b) 10 mM to 20 mM or 20 mM to 30 mM of the phosphate buffering agent, the acetate buffering agent, or the histidine salt buffering agent; (c) 0.2 mg/mL to 0.4 mg/mL of polysorbate 80 or polysorbate 20; and (d) 50 mg/mL to 60 mg/mL of sucrose, 50 mM to 60 mM of arginine or the pharmaceutically acceptable salt thereof, 200 mM to 350 mM or 250 mM to 350 mM of proline, and/or 10 mM to 30 mM or 10 mM to 20 mM of sodium chloride, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 30 mg/mL to 300 mg/mL of the anti-IL4Rα antibody or the antigen-binding fragment thereof; (b) 1 mM to 100 mM of the phosphate buffering agent or the histidine salt buffering agent; (c) 0.01 mg/mL to 2 mg/mL of polysorbate 80; and (d) 60 mM to 600 mM of proline and 1 mM to 80 mM of sodium chloride, and optionally, 10 mg/mL to 100 mg/mL of sucrose, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 50 mg/mL to 250 mg/mL of the anti-IL4Rα antibody or the antigen-binding fragment thereof; (b) 2 mM to 80 mM of the phosphate buffering agent or the histidine salt buffering agent; (c) 0.05 mg/mL to 1 mg/mL of polysorbate 80; and (d) 80 mM to 500 mM of proline and 2 mM to 60 mM of sodium chloride, and optionally, 20 mg/mL to 80 mg/mL of sucrose, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 70 mg/mL to 200 mg/mL of the anti-IL4Rα antibody or the antigen-binding fragment thereof; (b) 4 mM to 60 mM of the phosphate buffering agent or the histidine salt buffering agent; (c) 0.1 mg/mL to 0.8 mg/mL of polysorbate 80; and (d) 100 mM to 450 mM or 120 mM to 400 mM of proline and 4 mM to 50 mM of sodium chloride, and optionally, 30 mg/mL to 60 mg/mL of sucrose, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 100 mg/mL to 180 mg/mL or 120 mg/mL to 180 mg/mL of the anti-IL4Rα antibody or the antigen-binding fragment thereof; (b) 8 mM to 40 mM or 10 mM to 30 mM of the phosphate buffering agent or the histidine salt buffering agent; (c) 0.2 mg/mL to 0.6 mg/mL of polysorbate 80; and (d) 150 mM to 350 mM of proline and 8 mM to 40 mM of sodium chloride, and optionally, 40 mg/mL to 50 mg/mL of sucrose, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 120 mg/mL to 150 mg/mL or 150 mg/mL to 180 mg/mL of the anti-IL4Rα antibody or the antigen-binding fragment thereof; (b) 10 mM to 20 mM or 20 mM to 30 mM of the phosphate buffering agent or the histidine salt buffering agent; (c) 0.2 mg/mL to 0.4 mg/mL of polysorbate 80; and (d) 200 mM to 350 mM or 250 mM to 350 mM of proline and 10 mM to 30 mM or 10 mM to 20 mM of sodium chloride, and optionally, 50 mg/mL to 60 mg/mL of sucrose, wherein the pH of the pharmaceutical composition is 4 to 7, 5 to 7, 5 to 6.5, 5.3 to 6.5, 5.3 to 6.3, 5.3 to 5.8, or 5.8 to 6.3.

In some embodiments, the aforementioned pharmaceutical composition comprises: (a) 150 mg/mL of the anti-IL4Rα antibody or the antigen-binding fragment thereof; (b) 20 mM of the histidine-histidine hydrochloride buffering agent; (c) 0.4 mg/mL of polysorbate 80; and (d) 50 mg/mL of sucrose and 50 mM of arginine hydrochloride, wherein the pH of the pharmaceutical composition is 5.8.

In some embodiments, the aforementioned pharmaceutical composition comprises: (a) 150 mg/mL of the anti-IL4Rα antibody or the antigen-binding fragment thereof; (b) 20 mM of the histidine-histidine hydrochloride buffering agent; (c) 0.4 mg/mL of polysorbate 80; and (d) 250 mM of proline and 20 mM of sodium chloride, wherein the pH of the pharmaceutical composition is 5.8.

In some embodiments, the aforementioned pharmaceutical composition comprises: (a) 150 mg/mL of the anti-IL4Rα antibody or the antigen-binding fragment thereof; (b) 20 mM of the histidine-histidine hydrochloride buffering agent; (c) 0.4 mg/mL of polysorbate 80; and (d) 350 mM of proline, wherein the pH of the pharmaceutical composition is 5.8.

In some embodiments, the aforementioned pharmaceutical composition comprises: (a) 150 mg/mL of the anti-IL4Rα antibody or the antigen-binding fragment thereof; (b) 20 mM of the histidine-histidine hydrochloride buffering agent; (c) 0.4 mg/mL of polysorbate 80; and (d) 50 mg/mL of sucrose, 250 mM of proline, and 20 mM of sodium chloride, wherein the pH of the pharmaceutical composition is 5.8.

In an optional embodiment, the anti-IL4Rα antibody or the antigen-binding fragment thereof in the aforementioned pharmaceutical composition comprises a heavy chain variable region comprising a heavy chain variable region CDR1, a heavy chain variable region CDR2, and a heavy chain variable region CDR3, wherein the heavy chain variable region CDR1, the heavy chain variable region CDR2, and the heavy chain variable region CDR3 comprise amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 1, 2, and 3, respectively.

In an optional embodiment, the anti-IL4Rα antibody or the antigen-binding fragment thereof in the aforementioned pharmaceutical composition comprises a heavy chain variable region comprising an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, 8, or 9. The amino acid sequence of SEQ ID NO: 8 is: EVQLVESGGGLVQPGGSLRLSCAASGFTFSTYGMSWVRQAPGKGLVX$_1$VX$_2$TINSNGGST SYPDSVKGRFTISRDNAKNTLYLQMNSLRAEDTAVYYCARFFRFRNAMDYWGQGTLVTVS S, wherein, X$_1$ = W and X$_2$ = S, X$_1$ = L and X$_2$ = A, or X$_1$ = W and X$_2$ = A.

In an optional embodiment, the anti-IL4Rα antibody or the antigen-binding fragment thereof in the aforementioned pharmaceutical composition comprises a light chain variable region comprising a light chain variable region CDR1, a light chain variable region CDR2, and a light chain variable region CDR3, wherein the light chain variable region CDR1, the light chain variable region CDR2, and the light chain variable region CDR3 comprise amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 4, 5, and 6, respectively.

In an optional embodiment, the anti-IL4Rα antibody or the antigen-binding fragment thereof in the aforementioned pharmaceutical composition comprises a light chain variable region comprising an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 10, 11, or 12. The amino acid sequence of SEQ ID NO: 11 is: DIQMTQSPSSLSASVGDRVTITCRTSENIYSYLAWYQQKPGKAPKX$_1$LX$_2$YNAKTLAEGVPS RFSGSGSGTDFTLTISSLQPEDFATYYCQHYYGPPTWTFGQGTKVEIK, wherein, X$_1$ = L and X$_2$ = I, X$_1$ = F and X$_2$ = V, or X$_1$ = F and X$_2$ = I.

In an optional embodiment, the anti-IL4Rα antibody or the antigen-binding fragment thereof in the aforementioned pharmaceutical composition comprises a heavy chain variable region and a light chain variable region each comprising a CDR1, a CDR2, and a CDR3, wherein the heavy chain variable region CDR1, the heavy chain variable region CDR2, the heavy chain variable region CDR3, the light chain variable region CDR1, the light chain variable region CDR2, and the light chain variable region CDR3 comprise amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 1, 2, 3, 4, 5, and 6, respectively.

In an optional embodiment, the anti-IL4Rα antibody or the antigen-binding fragment thereof in the aforementioned pharmaceutical composition comprises a heavy chain variable region and a light chain variable region each comprising: (1) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 7 and 10, respectively; (2) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 8 and 11, respectively, wherein in SEQ ID NO: 8, X$_1$ = W and X$_2$ = S; (3) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 8 and 12, respectively, wherein in SEQ ID NO: 8, X$_1$ = W and X$_2$ = S; (4) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 9 and 11, respectively; (5) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 9 and 12, respectively; (6) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 8 and 11, respectively, wherein in SEQ ID NO: 8, X$_1$ = L and X$_2$ = A; (7) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 8 and 12, respectively, wherein in SEQ ID NO: 8, X$_1$ = L and X$_2$ = A; (8) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 8 and 11, respectively, wherein in SEQ ID NO: 8, X$_1$ = W and X$_2$ = A; or (9) amino acid sequences having at least 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 8 and 12, respectively, wherein in SEQ ID NO: 8, X$_1$ = Wand X$_2$ = A.

The amino acid sequence of SEQ ID NO: 8 is:

EVQLVESGGGLVQPGGSLRLSCAASGFTFSTYGMSWVRQAPGKGLVX$_1$VX$_2$TINSNGGST SYPDSVKGRFTISRDNAKNTLYLQMNSLRAEDTAVYYCARFFRFRNAMDYWGQGTLVTVS S;

the amino acid sequence of SEQ ID NO: 11 is:

DIQMTQSPSSLSASVGDRVTITCRTSENIYSYLAWYQQKPGKAPKX$_1$LX$_2$YNAKTLAEGVPS RFSGSGSGTDFTLTISSLQPEDFATYYCQHYYGPPTWTFGQGTKVEIK, wherein, X$_1$ = L and X$_2$ = I, X$_1$ = F and X$_2$ = V, or X$_1$ = F and X$_2$ = I.

In an optional embodiment, the anti-IL4Rα antibody or the antigen-binding fragment thereof in the aforementioned pharmaceutical composition comprises: a heavy chain comprising a heavy chain variable region and a heavy chain constant region, and a light chain comprising a light chain variable region and a light chain constant region, wherein the C terminus of the heavy chain variable region is linked to the N terminus of the heavy chain constant region and the C terminus of the light chain variable region is linked to the N terminus of the light chain constant region; the heavy chain variable region and the light chain variable region comprise the amino acid sequences described above; the heavy chain constant region is a human IgG4 constant region having the amino acid sequence set forth in SEQ ID NO: 13 and the light chain constant region is a human κ constant region having the amino acid sequence set forth in SEQ ID NO: 14.

In an optional embodiment, the anti-IL4Rα antibody or the antigen-binding fragment thereof in the aforementioned pharmaceutical composition may be a full-length antibody, e.g., an IgG1, IgG2, or IgG4 isotype full-length antibody, preferably an IgG4 isotype full-length antibody. In other embodiments, the anti-IL4Rα antibody or the antigen-binding fragment thereof in the aforementioned pharmaceutical composition may be a single chain variable fragment (scFv) antibody, or an antibody fragment, such as an Fab or F(ab')$_2$ fragment.

In an optional embodiment, the anti-IL4Rα antibody or the antigen-binding fragment thereof in the aforementioned pharmaceutical composition is the C2C1A1A1 antibody in Patent Application No. PCT/CN2021077784.

The present disclosure further provides a method for preparing the aforementioned pharmaceutical composition, comprising: contacting the aforementioned anti-IL4Rα antibody or the antigen-binding fragment thereof with a buffering agent, e.g., transferring the anti-IL4Rα antibody or the antigen-binding fragment thereof into a buffering agent including the phosphate buffering agent, the acetate buffering agent, or the histidine salt buffering agent, etc, and the method further comprises: adding a surfactant and a stabilizer without a specific order, wherein the stabilizer includes trehalose, mannitol, sucrose, arginine or the pharmaceutically acceptable salt thereof, proline and/or sodium chloride, and a surfactant includes a polysorbate, a poloxamer, polyethylene glycol, or a polyhydroxy compound.

The present disclosure further provides a method for preparing a lyophilized formulation comprising an anti-IL4Rα antibody or an antigen-binding fragment thereof, comprising: lyophilizing the aforementioned pharmaceutical composition. In some embodiments, the lyophilization is conducted by procedures well known in the art, including, but not limited to, pre-freezing, primary drying, and secondary drying. Those skilled in the art will appreciate that any method for removing water from the pharmaceutical composition of the present disclosure is suitable for use in the present disclosure.

The present disclosure further provides a lyophilized formulation comprising an anti-IL4Rα antibody or an antigen-binding fragment thereof prepared by the aforementioned method for preparing a lyophilized formulation.

The present disclosure further provides a lyophilized formulation comprising an anti-IL4Rα antibody or an antigen-binding fragment thereof capable of forming the aforementioned pharmaceutical composition upon reconstitution.

The present disclosure further provides an article of manufacture comprising a container comprising the aforementioned pharmaceutical composition or the aforementioned lyophilized formulation.

The pharmaceutical composition or the lyophilized formulation of the present disclosure may be administered according to known methods, e.g., by injection or infusion over a period of time in a suitable manner, e.g., by subcutaneous, intravenous, intraperitoneal, intramuscular, intraarterial, intralesional or intraarticular routes, topically, by inhalation or by sustained or delayed release. The pharmaceutical composition or the lyophilized formulation of the present disclosure may be diluted with a suitable diluent to an appropriate concentration prior to the administration to provide the optimal desired response (e.g., therapeutic response).

The present disclosure provides a method for reducing IL4 and/or IL13 signaling in a subject, comprising administering to the subject a therapeutically effective amount of the pharmaceutical composition, the lyophilized formulation, or the article of manufacture of the present disclosure. IL4 signals through receptors comprising IL-4Rα and γc, while IL13 signals through receptors comprising IL-4Rα and IL13 Rα1. The IL4 and/or IL13 signaling is manifested by the activation and/or proliferation of B cells, eosinophils and/or macrophages, the proliferation of fibroblasts, and the proliferation of smooth muscle cells (e.g., the proliferation of airway smooth muscle cells, etc.).

The present disclosure provides a method for reducing type 2 immune response in a subject, comprising: administering to the subject a therapeutically effective amount of the pharmaceutical composition, the lyophilized formulation, or the article of manufacture of the present disclosure.

The present disclosure provides a method for treating a disease associated with excessive IL4 and/or IL13 signaling in a subject, comprising: administering to the subject a therapeutically effective amount of the pharmaceutical composition, the lyophilized formulation, or the article of manufacture of the present disclosure. The disease associated with excessive IL4 and/or IL13 signaling is an allergic disease, of which the non-limiting examples include atopic dermatitis, anaphylaxis, allergic rhinitis, or allergic asthma.

The present disclosure provides a method for treating a tumor associated with increased STAT6 activation in a subject, comprising: administering to the subject a therapeutically effective amount of the pharmaceutical composition, the lyophilized formulation, or the article of manufacture of the present disclosure. In some embodiments, the tumor includes a solid tumor or a non-solid tumor. In some embodiments, examples of the tumor include, but are not limited to, melanoma, lung cancer, kidney cancer, prostate cancer, cervical cancer, colorectal cancer, gastric cancer, pancreatic cancer, ovarian cancer, and urothelial cancer.

Other features and advantages of the present disclosure will become apparent from the following detailed description and examples, which, however, should not be construed as limiting. The content of all of the documents, Genbank records, patents, and disclosed patent applications cited in the present disclosure is explicitly incorporated in the present disclosure by reference.

## SUMMARY

For a better understanding of the present disclosure, terms are defined herein. Other definitions are listed throughout the detailed description. Unless otherwise specifically defined herein, all other technical and scientific terms used herein have the same meaning as commonly understood by those of ordinary skills in the art to which the present disclosure belongs.

The "buffering agent" refers to a pharmaceutically acceptable substance or mixture of substances capable of maintaining the pH of a pharmaceutical composition in a desired pH range. Examples of buffering agents suitable for use in the pharmaceutical compositions of the present disclosure include phosphate buffering agents, acetate buffering agents, or histidine salt buffering agents.

The "phosphate buffering agent" is a buffering agent containing phosphate ions, e.g., a buffering agent containing phosphoric acid and/or a phosphate, wherein phosphoric acid includes phosphoric acid and/or a hydrate thereof, and the phosphate includes the phosphate and/or a hydrate thereof. Examples of the phosphate buffering agent include, but are not limited to, a sodium phosphate buffering agent or a potassium phosphate buffering agent, and preferably, the phosphate buffering agent is a sodium phosphate buffering agent. In one specific example, the sodium phosphate buffering agent contains sodium dihydrogen phosphate monohydrate ($NaH_2PO_4 \cdot H_2O$) and disodium hydrogen phosphate dodecahydrate ($Na_2HPO_4 \cdot 12H_2O$).

The "acetate buffering agent" is a buffering agent containing acetate ions, e.g., a buffering agent containing acetic acid and/or an acetate, wherein acetic acid includes acetic acid and/or a hydrate thereof, and the acetate includes the acetate and/or a hydrate thereof. Examples of the acetate buffering agent include, but are not limited to, a potassium acetate buffering agent, an ammonium acetate buffering agent, a sodium acetate buffering agent, or a sodium acetate-acetic acid buffering agent, and preferably, the acetate buffering agent is a sodium acetate-acetic acid buffering agent. The sodium acetate-acetic acid buffering agent contains acetic acid and sodium acetate, wherein acetic acid includes acetic acid and/or a hydrate thereof, and sodium acetate includes sodium acetate and/or a hydrate thereof. In one specific example, the sodium acetate-acetic acid buffering agent contains acetic acid and sodium acetate trihydrate ($CH_3COONa \cdot 3H_2O$).

The "histidine salt buffering agent" is a buffering agent containing histidine ions, e.g., a buffering agent containing histidine and/or a histidine salt, wherein histidine includes histidine and/or a hydrate thereof, and the histidine salt includes the histidine salt and/or a hydrate thereof. Examples of the histidine salt buffering agent include a histidine-hydrochloric acid buffering agent, a histidine-acetic acid buffering agent, a histidine-histidine hydrochloride, or a histidine hydrochloride buffering agent, and preferably, the histidine salt buffering agent is a histidine-histidine hydrochloride buffering agent. The histidine-histidine hydrochloride contains histidine and histidine hydrochloride, wherein histidine includes histidine and/or a hydrate thereof, and histidine hydrochloride includes histidine hydrochloride and/or a hydrate thereof. In one specific example, the histidine-histidine hydrochloride buffering agent includes histidine and histidine hydrochloride monohydrate ($C_6H_9N_3O_2 \cdot HCl \cdot H_2O$).

The "stabilizer" refers to a pharmaceutically acceptable substance or mixture of substances for maintaining the stability of the active ingredient in a pharmaceutical composition. In the present disclosure, the stabilizer may also be used as a viscosity reducer and/or an isotonizing agent, etc.

The "pharmaceutical composition" is intended to encompass a product comprising a particular active ingredient (e.g., an antibody), optionally in a particular amount, as well as any product that results, directly or indirectly, from combining particular active ingredients, optionally in particular amounts. The purpose of the pharmaceutical composition is to make the active ingredient suitable for production and administration to patients and to maintain biological activity and/or stability during storage and subsequent use. In some embodiments, the pharmaceutical composition is a water-soluble injection, including but not limited to a water-soluble formulation not lyophilized or a water-soluble formulation obtained by reconstituting a lyophilized powder. In some other embodiments, the pharmaceutical composition is a lyophilized formulation. In the present disclosure, the "pharmaceutical composition" and the "formulation" are not mutually exclusive.

The "stable" or "stabilized" pharmaceutical composition is a pharmaceutical composition in which an active ingredient (e.g., an antibody) substantially retains its physical and/or chemical stability and/or biological activity during storage. Various analytical techniques for determining the stability of an active ingredient are known in the art, for example, reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10:29-90 (1993). The stability can be measured at selected temperatures and in other storage conditions over a selected period of time. For example, an active ingredient "retains its physical stability" in a pharmaceutical composition if it does not exhibit a significant increase in aggregation, precipitation and/or denaturation upon visual inspection of color and/or clarity, or as measured by UV light scattering, size exclusion chromatography (SEC), differential scanning calorimetry (DSC), or differential scanning fluorimetry (DSF).

Preferably, when the pharmaceutical composition of the present disclosure is used, 10% or less, 5% or less, or 4% or less of the active ingredient forms aggregates (also called macromolecular impurities), as measured by, for example, SEC or any other suitable method for measuring aggregate formation. An active ingredient (e.g., an antibody) "retains its chemical stability" in a pharmaceutical composition if the active ingredient does not exhibit a significant chemical change. The chemical stability can be assessed by detecting and quantifying the chemically altered formats of the antibody. The processes that often alter the chemical structure of a protein include hydrolysis or truncation (as assessed by methods such as size exclusion chromatography and SDS-PAGE), oxidation (as assessed by methods such as peptide mapping coupled with mass spectrometry or MALDI/TOF/MS), deamidation (as assessed by methods such as ion exchange chromatography, capillary isoelectric focusing, peptide mapping, and isoaspartic acid measurement), and isomerization (as assessed by measuring isoaspartic acid content, by peptide mapping, etc.). An active ingredient (e.g., an antibody) "retains its biological activity" for a given period in a pharmaceutical composition, as determined, for example, by an antigen binding assay, if the biological activity of the active ingredient for the given period is within a predetermined range of the biological activity exhibited when the pharmaceutical composition is prepared.

The "macromolecular impurities" or "aggregates" refer to the generic term for impurities having a molecular weight greater than that of the active ingredient of interest (e.g., an antibody).

The "charge variant" refers to variants of an antibody that undergo glycosylation, deamidation, oxidation, and/or isomerization, etc., and that directly or indirectly cause changes in the charges of the antibody molecules. These charge variants can be detected by capillary isoelectric focusing electrophoresis (CIEF) and cation exchange chromatography (CEX-HPLC), etc.

The articles "a", "an" and "the" are used herein to refer to one or more (i.e., at least one) of the grammatical objects of the article. For example, "a pharmaceutical composition" refers to one pharmaceutical composition or more than one pharmaceutical composition.

The term "about" or "approximately" means that a numerical value is within an acceptable error range for the particular value determined by those of ordinary skills in the art, and the numerical value depends in part on how the measurement or determination is carried out (i.e., the limits of the measurement system). For example, "about" or "approximately" in the art can mean a standard deviation within 1 or exceeding 1. Alternatively, "about" or "approximately" represents a range of $\pm 15\%$, $\pm 10\%$, $\pm 5\%$ or $\pm 1\%$. Furthermore, particularly for a biological system or process, the term may refer to at most one order of magnitude or at most 5 times the value. Unless otherwise stated, "about XX", "approximately XX", or "substantially comprising XX" refers to a numeral value within an acceptable error range for the particular value "XX" (including the numeral value "XX" itself, as well as those within an acceptable error range for the determination of the numeral value by those of ordinary skills in the art).

As described herein, any proportion range, ratio range, or integer range shall be construed as including the value of any integer within the listed range and including, when appropriate, fractions thereof (such as one tenth and one hundredth of the integer) unless otherwise indicated.

In the present disclosure, unless the context dictates otherwise, the words "comprise", "include" and "contain" will be interpreted as including the steps or elements or a group of steps or elements but not excluding any other steps or elements or groups of steps or elements. "Consisting of..." means including and being limited to what follows the phrase "consisting of...". Thus, the phrase "consisting of..." means that the listed elements are required or necessary and that no other elements can be present. "Substantially consisting of..." means including any listed element that follows the phrase and being limited to other elements that do not interfere with or are favorable for the listed elements' activity or effects as detailed in the present disclosure. Thus, the phrase "substantially consisting of..." means that the listed elements are required or necessary but other elements are optional and can be present or absent depending on whether they affect the listed elements' activity or effects.

The term "IL4Rα" refers to interleukin 4 receptor α subunit. The term "IL4Rα" includes variants, isoforms, homologs, orthologs, and paralogs. For example, in certain instances, an antibody specific for human IL4Rα protein may cross-react with IL4Rα protein from a species other than a human (e.g., monkey). In other embodiments, an antibody specific for a human IL4Rα protein can be completely specific for the human IL4Rα protein and does not cross-react with proteins of other species or other types, or can cross-react with IL4Rα derived from some other species but not all others.

The term "human IL4Rα" refers to an IL4Rα protein having a human amino acid sequence, e.g., the amino acid sequence of human IL4Rα with Genbank Accession No. NP_001244335.1. The terms "cynomolgus monkey IL4Rα" and "marmoset monkey IL4Rα" refer to IL4Rα sequences having, for example, the amino acid sequences with Genbank Accession Nos. EHH60265.1 and NP_001244161.1, respectively.

The term "antibody" herein includes full-length antibodies and any antigen-binding fragment or single chain thereof. The full-length antibody is a glycoprotein comprising two heavy (H) chains and two light (L) chains linked by disulfide bonds. Each of the heavy chains is composed of a heavy chain variable region (abbreviated as $V_H$) and a heavy chain constant region. The heavy chain constant region is composed of three domains: $C_{H1}$, $C_{H2}$, and $C_{H3}$. Each of the light chains is composed of a light chain variable region (abbreviated as $V_L$) and a light chain constant region. The light chain constant region is composed of one domain $C_L$. The $V_H$ and $V_L$ regions may also be divided into hypervariable regions, known as complementarity determining regions (CDRs), which are separated by more conservative framework regions (FRs). $V_H$ and $V_L$ are each composed of three CDRs and four FRs, arranged from the amino-terminus to the carboxyl-terminus in the following order: FR1, CDR1, FR2, CDR2, FR3, CDR3, FR4. The variable regions of the heavy chains and light chains comprise binding domains that interact with antigens. The constant regions of the antibody can mediate the binding of immunoglobulins to host tissues or factors, including various cells of the immune system (e.g., effector cells) and the first component (C1q) of the classical complement system.

As used herein, the "antigen-binding fragment" of an antibody (or abbreviated as "antibody fragment"), refers to one or more fragments of the antibody that retain the ability to specifically bind to an antigen (e.g., IL4Rα protein). It has been demonstrated that the antigen-binding functionality of an antibody can be implemented by fragments of a full-length antibody. Examples of binding fragments encompassed within the "antigen-binding fragment" of an antibody include: (i) an Fab fragment, a monovalent fragment composed of $V_L$, $V_H$, $C_L$, and $C_{H1}$ domains; (ii) an F(ab')$_2$ fragment, a bivalent fragment comprising two Fab fragments linked by a disulfide bond at the hinge region; (iii) an Fd fragment composed of $V_H$ and $C_{H1}$; (iv) an Fv fragment composed of $V_L$ and $V_H$ of a single arm of the antibody; (v) a dAb fragment composed of $V_H$ (Ward et al., (1989) Nature 341:544-546); (vi) isolated complementarity determining regions (CDRs); and (vii) a nanobody, a heavy chain variable region comprising a single variable domain and two constant domains. Furthermore, although the two domains $V_L$ and $V_H$ of the Fv fragment are encoded by different genes, they can be joined via a synthetic linker by recombinant means into a single protein chain in which $V_L$ and $V_H$ pair to form a monovalent molecule (referred to as single-chain Fv (scFv); see, e.g., Bird et al., (1988) Science 242:423-426; and Huston et al., (1988) Proc. Natl. Acad. Sci. USA 85:5879-5883). Such single-chain antibodies are also encompassed within the term "antigen-binding fragment" of the antibody. These antibody fragments can be obtained using conventional techniques known to those skilled in the art, and the fragments can be subjected to functional screening using the same method as full-length antibodies.

As used herein, the term "isolated antibody" refers to an antibody that is substantially free of other antibodies having different antigenic specificities (e.g., an isolated antibody that specifically binds to IL4Rα protein is substantially free of antibodies that specifically bind to antigens other than IL4Rα protein). However, an isolated antibody that specifically binds to human IL4Rα protein may be cross-reactive to other antigens, such as IL4Rα protein of other species. Furthermore, the isolated antibody is substantially free of other cellular materials and/or chemical substances.

As used herein, the term "mouse antibody" refers to an antibody in which the framework regions and CDRs in the variable region are derived from mouse germline immunoglobulin sequences. Furthermore, if the antibody comprises a constant region, the constant region is also derived from mouse germline immunoglobulin sequences. The mouse antibody in the present disclosure may comprise amino acid residues not encoded by mouse germline immunoglobulin sequences (e.g., mutations introduced by *in-vitro* random or point mutations, or by *in-vivo* somatic mutations). However, the term "mouse antibody" as used herein does not include antibodies in which CDR sequences from other mammalian species are inserted between the mouse framework region sequences.

The "chimeric antibody" refers to an antibody obtained by combining genetic substances of non-human origin with genetic substances of human origin. More generally, a chimeric antibody refers to an antibody that combines genetic substances of one species with genetic substances of another species.

As used herein, the term "humanized antibody" refers to an antibody from a non-human species of which the protein sequence has been modified to increase its similarity to a natural human antibody. In some specific examples, the "humanized antibody" comprises complementarity determining regions (CDRs) derived from a non-human antibody, and framework regions (FRs) and constant regions derived from a human antibody.

The term "isotype" refers to the class of antibodies encoded by the heavy chain constant region genes (e.g., IgM or IgG1).

The phrases "antigen-recognizing antibody" and "antibody specific to an antigen/antibody having specificity to an antigen" are used herein interchangeably with the term "antibody specifically binding to an antigen".

As used herein, the antibody "specifically binding to human IL4Rα" refers to an antibody that binds to human IL4Rα protein (and possibly to IL4Rα protein of one or more non-human species) but substantially does not bind to non-IL4Rα proteins. Preferably, the antibody binds to a human IL4Rα with "high affinity", i.e., binds to a human IL4Rα with a $K_D$ value of $5.0 \times$ $^{-8}$ M or less, preferably $1.0 \times 10^{-8}$ M or less, and more preferably $7.0 \times 10^{-9}$ M or less.

As used herein, the term "substantially not bind to" a protein or cell refers to not binding to the protein or cell, or not binding to the protein or cell with high affinity, i.e., binding to the protein or cell with a $K_D$ value of $1.0 \times 10^{-6}$ M or greater, preferably $1.0 \times 10^{-5}$ M or greater, more preferably $1.0 \times 10^{-4}$ M or greater, even more preferably $1.0 \times 10^{-3}$ M or greater, and still more preferably $1.0 \times 10^{-2}$ M or greater.

The term "high affinity" for IgG antibodies refers to that the antibody has a $K_D$ value for the antigen of $1.0 \times 10^{-6}$ M or less, preferably $5.0 \times 10^{-8}$ M or less, more preferably $1.0 \times 10^{-8}$ M or less, even more preferably $7.0 \times 10^{-9}$ M or less, and still more preferably $1.0 \times 10^{-9}$ M or less. However, for other antibody isotypes, the "high affinity" binding may be different. For example, for IgM isotype, the "high affinity" binding refers to that the antibody has a $K_D$ value of

$1.0 \times 10^{-6}$ M or less, preferably $1.0 \times 10^{-7}$ M or less, and more preferably $1.0 \times 10^{-8}$ M or less.

As used herein, the term "$K_{assoc}$" or "$K_a$" refers to the association rate of a particular antibody-antigen interaction, and the term "$K_{dis}$" or "$K_d$" refers to the dissociation rate of a particular antibody-antigen interaction. As used herein, the term "$K_D$" refers to the dissociation constant, which is the ratio of $K_d$ to $K_a$ (i.e., $K_d/K_a$) and is expressed in molar concentration (M). The $K_D$ value of an antibody can be determined using methods well known in the art. A preferred method for determining the $K_D$ value of an antibody comprises using surface plasmon resonance, preferably using a biosensor system, such as the Biacore™ system.

The term "$EC_{50}$", also known as half-maximal effective concentration, refers to the concentration of an antibody that induces a response halfway between the baseline and the maximum after a particular exposure time. The term "$IC_{50}$", also known as half-maximal inhibitory concentration, refers to the concentration of an antibody that inhibits a specific biological or biochemical function by 50% relative to the case where the antibody is absent.

The term "subject" includes any human or non-human animal. The term "non-human animal" includes all vertebrates, such as mammals and non-mammals, such as non-human primates, sheep, dogs, cats, cows, horses, chickens, amphibians, and reptiles; mammals such as non-human primates, sheep, dogs, cats, cows, and horses are preferred.

The term "therapeutically effective amount" refers to an amount of the antibody of the present disclosure sufficient to prevent or ameliorate symptoms related to diseases or conditions (e.g., cancers), and/or reduce the severity of diseases or conditions. It should be understood that the therapeutically effective amount is related to the disease to be treated, wherein the actual effective amount can be readily determined by those skilled in the art.

The term "identity" refers to the sequence similarity between two polynucleotide sequences or between two polypeptides. Sequence comparisons between two sequences and percentage identity determination can be conducted by the BLASTN/BLASTP algorithm on the website of the U.S. National Center for Biotechnology Information with default settings.

The terms "Xn" and "Xaa" are equivalent and refer to an unspecified amino acid whose scope is specified by the subsequent definitions in the relevant description.

Aspects of the present disclosure are described in more detail below.

Anti-IL4Rα antibody or antigen-binding fragment thereof

The anti-IL4Rα antibody or the antigen-binding fragment thereof in the present disclosure specifically binds to human IL4Rα with comparable, if not better, binding affinity/capacity than reported anti-IL4Rα antibodies (e.g., dupilumab).

The anti-IL4Rα antibody or the antigen-binding fragment thereof in the present disclosure is capable of blocking the binding of IL4Rα to IL4 or IL13-IL13Rα1, thereby blocking the corresponding intracellular signal transduction, with comparable or superior blocking activity to reported anti-IL4Rα antibodies (e.g., dupilumab).

Preferably, the anti-IL4Rα antibody of the present disclosure is a humanized monoclonal antibody. Additionally or alternatively, the anti-IL4Rα antibody of the present disclosure may be, for example, a mouse, chimeric, or human monoclonal antibody.

The anti-IL4Rα antibody in the present disclosure is a monoclonal antibody with structural and chemical properties as described below and in the examples. The amino acid sequence IDs of the heavy/light chain variable regions of the antibodies are summarized in Table 1 below. Some antibodies have the same $V_H$ or $V_L$. The heavy chain constant region of the antibody may be a human IgG4 heavy chain constant region having the amino acid sequence set forth in SEQ ID NO: 13, and the light chain constant region of the antibody may be a human κ constant region having the amino acid sequence set forth in SEQ ID NO: 14.

Table 1. Amino acid sequence IDs of heavy/light chain variable regions

| Antibody | VH-CDR 1 | VH-CDR 2 | VH-CDR3 | VH | VL-CDR1 | VL-CDR2 | VL-CDR 3 | VL |
|---|---|---|---|---|---|---|---|---|
| C2C1A1A1 | | | | 7 | | | | 10 |
| huC2C1A1A1 -V1 | 1 | 2 | 3 | 8, $X_1$=W, $X_2$=S | 4 | 5 | 6 | 11, $X_1$=L, $X_2$=I |

(continued)

| Antibody | VH-CDR 1 | VH-CDR 2 | VH-CDR3 | VH | VL-CDR1 | VL-CDR2 | VL-CDR 3 | VL |
|---|---|---|---|---|---|---|---|---|
| huC2C1A1A1 -V2 | | | | 8, $X_1$=W, $X_2$=S | | | | 12 |
| huC2C1A1A1 -V3 | | | | 8, $X_1$=W, $X_2$=S | | | | 11, $X_1$=F, $X_2$=V |
| huC2C1A1A1 -V4 | | | | 8, $X_1$=W, $X_2$=S | | | | 11, $X_1$=F, $X_2$=I |
| huC2C1A1A1 -V5 | | | | 9 | | | | 11, $X_1$=L, $X_2$=I |
| huC2C1A1A1 -V6 | | | | 9 | | | | 12 |
| huC2C1A1A1 -V7 | | | | 9 | | | | 11, $X_1$=F, $X_2$=V |
| huC2C1A1A1 -V8 | | | | 9 | | | | 11, $X_1$=F, $X_2$=I |
| huC2C1A1A1 -V9 | | | | 8, $X_1$=L, $X_2$=A | | | | 11, $X_1$=L, $X_2$=I |
| huC2C1A1A1 -V10 | | | | 8, $X_1$=L, $X_2$=A | | | | 12 |
| huC2C1A1A1 -V11 | | | | 8, $X_1$=L, $X_2$=A | | | | 11, $X_1$=F, $X_2$=V |
| huC2C1A1A1 -V12 | | | | 8, $X_1$=L, $X_2$=A | | | | 11, $X_1$=F, $X_2$=I |
| huC2C1A1A1 -V13 | | | | 8, $X_1$=W, $X_2$=A | | | | 11, $X_1$=L, $X_2$=I |
| huC2C1A1A1 -V14 | | | | 8, $X_1$=W, $X_2$=A | | | | 12 |
| huC2C1A1A1 -V15 | | | | 8, $X_1$=W, $X_2$=A | | | | 11, $X_1$=F, $X_2$=V |
| huC2C1A1A1 -V16 | | | | 8, $X_1$=W, $X_2$=A | | | | 11, $X_1$=F, $X_2$=I |

The heavy chain variable region CDRs and light chain variable region CDRs in Table 1 are defined by the Kabat numbering system. However, as is well known in the art, the CDRs may also be determined by other numbering systems such as the Chothia, IMGT, AbM, or Contact numbering system/method based on the heavy/light chain variable region sequence. Where the antibody or the antigen-binding fragment thereof is defined by particular CDR sequences, the scope of the antibody encompasses antibodies or antigen-binding fragments thereof defined by CDR sequences defined by any numbering system in the art (e.g., Chothia, IMGT, AbM, or Contact numbering system).

$V_H$ and $V_L$ sequences (or CDR sequences) of other anti-IL4Rα antibodies that bind to human IL4Rα may be "mixed and paired" with $V_H$ and $V_L$ sequences (or CDR sequences) of the anti-IL4Rα antibody in the present disclosure. Preferably, when $V_H$ and $V_L$ chains (or CDRs in these chains) are mixed and paired, the $V_H$ sequence in a particular $V_H/V_L$ pair is substituted with a structurally similar $V_H$ sequence. Likewise, it is preferred to substitute the $V_L$ sequence in a particular $V_H/V_L$ pair with a structurally similar $V_L$ sequence.

Therefore, in one embodiment, the anti-IL4Rα antibody or the antigen-binding fragment thereof in the present disclosure comprises:

(a) a heavy chain variable region comprising an amino acid sequence listed in Table 1; and
(b) a light chain variable region comprising an amino acid sequence listed in Table 1, or the $V_L$ of another anti-IL4Rα antibody, wherein the antibody or the antigen-binding fragment thereof specifically binds to human IL4Rα.

In another embodiment, the anti-IL4Ra antibody or the antigen-binding fragment thereof in the present disclosure comprises:

(a) the CDR1, CDR2, and CDR3 of a heavy chain variable region listed in Table 1; and
(b) the CDR1, CDR2, and CDR3 of a light chain variable regions listed in Table 1, or the CDRs of another anti-IL4Rα antibody, wherein the antibody or the antigen-binding fragment thereof specifically binds to human IL4Rα.

In another embodiment, the anti-IL4Ra antibody or the antigen-binding fragment thereof in the present disclosure comprises the CDR2 of the heavy chain variable region of the anti-IL4Rα antibody of the present disclosure and the CDRs of other antibodies that bind to human IL4Rα, e.g., the CDR1 and/or CDR3 from the heavy chain variable region, and/or the CDR1, CDR2 and/or CDR3 from the light chain variable region of another anti-IL4Rα antibody.

In addition, it is well known in the art that, independent of the CDR1 and/or CDR2 domains, the CDR3 domain can individually determine the binding specificity of an antibody to a homologous antigen, and that multiple antibodies with the same binding specificity can be predictively generated based on a common CDR3 sequence.

Accordingly, in another embodiment, the anti-IL4Rα antibody or the antigen-binding fragment thereof in the present disclosure comprises the CDR2 of the heavy chain variable region of the anti-IL4Rα antibody of the present disclosure and at least the CDR3 of the heavy chain variable region and/or the light chain variable region of the anti-IL4Ra antibody of the present disclosure, or the CDR3 of the heavy chain variable region and/or the light chain variable region of another anti-IL4Rα antibody, wherein the antibody specifically binds to human IL4Rα. Such antibodies preferably (a) compete with the anti-IL4Rα antibody of the present disclosure for binding to IL4Rα; (b) retain the functional properties of the anti-IL4Rα antibody of the present disclosure; (c) bind to the same epitope as the anti-IL4Rα antibody of the present disclosure; and/or (d) have similar binding affinities to the anti-IL4Ra antibodies of the present disclosure. In another embodiment, the anti-IL4Rα antibody of the present disclosure may further comprise the CDR2 of the light chain variable region of the anti-IL4Rα antibody of the present disclosure, or the CDR2 of the light chain variable region of another anti-IL4Rα antibody, wherein the antibody specifically binds to human IL4Rα. In another embodiment, the anti-IL4Rα antibody of the present disclosure may further comprise the CDR1 of the heavy chain variable region and/or the light chain variable region of the anti-IL4Rα antibody of the present disclosure, or the CDR1 of the heavy chain variable region and/or the light chain variable region of another anti-IL4Rα antibody, wherein the antibody specifically binds to human IL4Rα.

In another embodiment, the anti-IL4Ra antibody or the antigen-binding fragment thereof in the present disclosure comprises a heavy chain variable region and/or a light chain variable region each comprising a CDR1, a CDR2, and a CDR3, wherein the sequences of the CDR1, the CDR2, and the CDR3 are different from the sequences of the CDR1, the CDR2, and the CDR3 of the anti-IL4Rα antibody in the present disclosure, and the difference is derived from one or more conservative modifications. It will be appreciated in the art that some conservative sequence modifications do not eliminate the antigen-binding ability. See, e.g., Brummell et al., (1993) Biochem 32:1180-8; de Wildt et al., (1997) Prot. Eng. 10:835-41; Komissarov et al., (1997) J. Biol. Chem. 272:26864-26870; Hall et al., (1992) J. Immunol. 149:1605-12; Kelley and O'Connell (1993) Biochem. 32:6862-35; Adib-Conquy et al., (1998) Int. Immunol. 10:341-6 and Beers et al., (2000) Clin. Can. Res. 6:2835-43.

Accordingly, in one embodiment, the anti-IL4Rα antibody or the antigen-binding fragment thereof in the present disclosure comprises a heavy chain variable region and/or a light chain variable region each comprising a CDR1, a CDR2, and a CDR3, wherein:

(a) the CDR1 of the heavy chain variable region comprises a sequence listed in Table 1, and/or conservative modifications thereof; and/or

(b) the CDR2 of the heavy chain variable region comprises a sequence listed in Table 1, and/or conservative modifications thereof; and/or

(c) the CDR3 of the heavy chain variable region comprises a sequence listed in Table 1, and/or conservative modifications thereof; and/or

(d) the CDR1 and/or the CDR2 and/or the CDR3 of the light chain variable region comprise sequences listed in Table 1, and/or conservative modifications thereof; and

(e) the antibody or the antigen-binding fragment thereof specifically binds to human IL4Rα. As used herein, the term "conservative sequence modification" refers to an amino acid modification that does not significantly affect or alter the binding characteristic of the antibody. Such conservative modifications include amino acid substitutions, additions, and deletions. Modifications can be introduced into the antibody in the present disclosure using standard techniques known in the art, e.g., point mutation and PCR-mediated mutation. Conservative amino acid substitutions are those in which an amino acid residue is replaced by an amino acid residue having a similar side chain. Amino acid residue groups having similar side chains are known in the art. Such amino acid residue groups include amino acids with basic side chains (e.g., lysine, arginine, and histidine), amino acids with acidic side chains (e.g., aspartic acid, and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with β-branched side chains (e.g., threonine, valine, and isoleucine), and amino acids with aromatic side chains (e.g., tyrosine, phenylalanine, tryptophan, and histidine). Thus, one or more amino acid residues in a CDR region of the anti-IL4Rα antibody in the present disclosure may be replaced by other amino acid residues from the same side chain group, and the resultant antibody may be tested for function retention (i.e., the function described above) by the function assays described in the present disclosure.

The pharmaceutical composition, the lyophilized formulation, or the article of manufacture of the present disclosure have wide *in vitro* and *in vivo* use, relating to, for example, the treatment of a disease associated with excessive IL4 and/or IL13 signaling.

In one aspect, anti-IL4Rα antibodies or antigen-binding fragments thereof can block the binding of IL4Rα to IL4 or IL13-IL13Rα1, so as to reduce type 2 immunity. The present disclosure provides a method for treating an allergic disease associated with type 2 immunity in a subject, comprising: administering to the subject the pharmaceutical composition, the lyophilized formulation, or the article of manufacture of the present disclosure. The allergic disease may be atopic dermatitis, anaphylaxis, allergic rhinitis, or allergic asthma.

In another aspect, IL4 or IL13 signaling can activate STAT6, and STAT6 inhibitors have been found to be capable of inhibiting cancer cell growth. The present disclosure provides a method for inhibiting tumor cell growth in a subject, comprising: administering to the subject the pharmaceutical composition, the lyophilized formulation, or the article of manufacture of the present disclosure. Non-limiting examples of the tumor include, but are not limited to, melanoma, lung cancer, kidney cancer, prostate cancer, cervical cancer, colorectal cancer, gastric cancer, pancreatic cancer, ovarian cancer, and urothelial cancer.

In another aspect, the present disclosure provides a method for reducing or inhibiting cell activation in response to IL-4 or IL-13 in a subject. In some embodiments, the inhibition of activation comprises inhibiting the production or secretion of a cytokine. In some embodiments, the inhibition of activation comprises inhibiting proliferation. Cells that respond to IL-4 by activating the hybrid IL-4Rα/γc receptor include, but are not limited to, B cells, eosinophils, and macrophages. Cells that respond to IL-13 by activating the hybrid IL-4Rα/IL-13Rα1 receptor include, but are not limited to, fibroblasts and smooth muscle cells. Accordingly, in one embodiment, the present disclosure provides a method for inhibiting smooth muscle cell proliferation. In another embodiment, the present disclosure provides a method for inhibiting fibroblast proliferation.

The present disclosure is further illustrated by the following examples, which should not be construed as limiting. All of the drawings of the present disclosure and all of the references, Genebank sequences, patents, and disclosed patent disclosure cited herein are explicitly incorporated herein by reference.

**Example**

The preparation and purification methods of the anti-IL4Rα antibody in the present disclosure are described in Patent Application No. PCT/CN2021077784, which is incorporated herein by reference in its entirety.

**Example 1** Affinity assay of anti-IL4Rα antibodies by BIACORE surface plasmon resonance

The binding affinity and binding kinetics of the anti-IL4Rα antibodies (mAbs) in the present disclosure were characterized by a Biacore T200 system (GE Healthcare, Pittsburgh, PA, USA).

Briefly, a goat anti-human IgG (GE Healthcare, Cat. No. BR100839, Human Antibody Capture Kit) was covalently attached to a CM5 chip (carboxymethylated dextran-coated chip) via the primary amine group using a standard amine coupling kit supplied by Biacore (GE Healthcare, Pittsburgh, PA, USA). Unreacted portions on the biosensor surface were blocked with ethanolamine. Then, the anti-IL4Rα antibody in the present disclosure at 66.67 nM and an anti-IL4Rα antibody benchmark (dupilumab, or DUPIXENT® or BM) at 10 μg/mL were directed through the chip at a flow rate of 10 μL/min. A recombinant human IL4Rα-his protein (prepared in-house, amino acid sequence set forth in SEQ ID NO: 15), a cynomolgus monkey IL4Rα-his protein (Sino Biological Inc., Cat. No. 90897-C08H), or a marmoset monkey IL4Rα-his protein (customized product from Sino Biological Inc., denoted as cal-IL4Rα-his, amino acid sequence set forth in SEQ ID NO: 16) serially diluted with an HBS EP buffer (supplied by Biacore) was directed through the chip at a flow rate of 30 μL/min. The antigen-antibody binding kinetics was tracked for 2 min and the dissociation kinetics was tracked for 10 min. The association and dissociation curves were fitted according to a 1:1 Langmuir binding model using BIA evaluation software, and the $K_D$, $K_a$, and $K_d$ values were determined.

**Example 2** Binding activity of anti-IL4Rα antibodies to IL4Rα

The binding activity of the anti-IL4Rα antibodies in the present disclosure to IL4Rα was determined by capture ELISA, flow cytometry (FACS), and indirect ELISA.

*2.1 Capture ELISA*

Briefly, an affinity-purified goat anti-human IgG (Fcγ fragment-specific, Jackson Immunoresearch, Cat. No. 109-005-098) in PBS at a concentration of 2 μg/mL was immobilized on a 96-well plate at 100 μL/well and incubated overnight at 4 °C. The plate was washed once with a washing buffer (PBS + 0.05% w/v Tween-20, PBST) and then blocked at 37 °C for 2 h by adding 200 μL/well of a blocking buffer (PBST containing 5% w/v skim milk). The plate was washed again, and the anti-IL4Rα antibodies in the present disclosure, the benchmark, or the negative control hIgG (human immunoglobulin for intravenous injection (pH 4), Hualan Biological Engineering Inc.) serially 5-fold diluted from 66.7 nM in a PBST containing 2.5% w/v of skim milk was added at 100 μL per well. The plate was incubated for 40 min at 37 °C and washed 4 times. A biotinylated human IL4Rα-his protein (prepared in-house, amino acid sequence set forth in SEQ ID NO: 15, dissolved in a PBST containing 2.5% w/v skim milk at a concentration of 0.14 nM) was added at 100 μL/well to a 96-well plate coated with the anti-IL4Rα antibodies. The plate was incubated at 37 °C for 40 min and washed 4 times. An HRP-labeled streptavidin (diluted at 1:10000 in PBST, Jackson Immuno Research, Cat. No. 016-030-084) was added at 100 μL/well, and the plate was incubated at 37 °C for another 40 min. After a final wash, 100 μL/well of ELISA substrate TMB (Innoreagents, Cat. No. TMB-S-002) was added and the plate was incubated. After 10 min, the reaction was stopped by adding 50 μL/well of 1 M $H_2SO_4$ at 25 °C and the absorbance at 450 nm was measured. Data were analyzed using Graphpad Prism software and $EC_{50}$ values were reported.

*2.2 Cell-based binding FACS*

The binding activity of the anti-IL4Rα antibodies in the present disclosure to IL4Rα expressed on the surface of 293F-IL4Rα cells was determined by flow cytometry (FACS). Briefly, 293F cells (Thermofisher Inc., Cat. No. 11625019) were transfected with a pCMV-T-P plasmid construct having nucleotides encoding human IL4Rα (amino acid residues 1-825 of Uniprot # P24394-1) between EcoRI and XbaI. A stable cell pool (designated as 293F-IL4Rα) was selected for subsequent cell-based binding FACS and cell-based ligand blocking FACS analysis. The 293F-IL4Rα cells were harvested from cell culture flasks, washed twice, and resuspended in an FACS buffer (phosphate buffer saline (PBS) containing 2% v/v of fetal bovine serum). Then, the anti-IL4Rα antibodies or control serially 4-fold diluted in the FACS buffer from 80 nM was added at 100 μL/well to a 96-well plate containing $2 \times 10^5$ cells/well. The plate was incubated in an ice bath for 40 min. After the cells were washed twice with the FACS buffer, R-phycoerythrin affinipure goat anti-human IgG (Fcγ fragment-specific, Jackson Immunoresearch, Cat. No. 109-115-098, 1:1000 diluted in the FACS buffer) was added at 100 μL/well. After an incubation at 4 °C for 40 min in the dark, the cells were washed thrice and resuspended in the FACS buffer. The fluorescence values were measured using Becton Dickinson FACS Canto II-HTS. Data were analyzed using Graphpad Prism software and $EC_{50}$ values were reported.

*2.3 Indirect ELISA*

The anti-IL4Rα antibodies in the present disclosure were tested for cross-reactivity with cynomolgus monkey IL4Rα protein or cal-IL4Rα-his protein. Briefly, cynomolgus monkey IL4Rα-his protein (Sino Biological Inc., Cat. No. 90897-C08H) in a carbonate/bicarbonate buffer (pH 9.6) at 2 μg/mL or cal-IL4Rα-his protein (Sino Biological Inc.,

customized product, Cat. No. BAX2) in a carbonate/bicarbonate buffer (pH 9.6) at 0.2 $\mu$g/mL was immobilized on a 96-well plate at 100 $\mu$L/well, and incubated at 37 °C for 2 h. The plate was washed once with a washing buffer (PBS + 0.05% w/v Tween-20, PBST) and then blocked at 37 °C for 2 h by adding 200 $\mu$L/well of a blocking buffer (PBST containing 5% w/v skim milk). The plate was washed again, and the anti-IL4Ra antibodies in the present disclosure or control serially 5-fold diluted from 66.7 nM to 0.004 nM in a PBST containing 2.5% w/v of skim milk was added at 100 $\mu$L per well. The plate was incubated for 40 min at 37 °C. The plates were washed 4 times, and Peroxidase affiniPure F(ab')$_2$ fragment goat anti-human IgG (Fc$\gamma$ fragment-specific, Jackson Immunoresearch, Cat. No. 109-036-098, 1:5000 diluted in a PBST buffer) was added at 100 $\mu$L/well. The plate was incubated for 40 min at 37 °C. After a final wash, the plate was added with TMB (Innoreagents) at 100 $\mu$L/well for incubation. After 3-10 min, the reaction was stopped by adding 50 $\mu$L/well of 1 M H$_2$SO$_4$ at 25 °C and the absorbance at 450 nm was measured. Data were analyzed using Graphpad Prism software and EC$_{50}$ values were reported.

**Example 3** Blocking activity of anti-IL4R$\alpha$ antibodies on IL4R$\alpha$-benchmark or IL4R$\alpha$-IL4 interaction

*3.1 Ligand blocking ELISA*

The capacity of the anti-IL4R$\alpha$ antibodies in the present disclosure to block the IL4-IL4R$\alpha$ interaction was determined by competitive ELISA. Briefly, human IL4R$\alpha$-his protein (prepared in-house, amino acid sequence set forth in SEQ ID NO: 15) in PBS at 2 $\mu$g/mL was immobilized on a 96-well plate at 100 $\mu$L/well and incubated overnight at 4 °C. The next day, the plate was washed with a washing buffer (PBS + 0.05% w/v Tween-20, PBST) before a PBST containing 5% w/v of skim milk was added. The plate was then incubated at 37 °C for 2 h for blocking. The plate was then washed again with the washing buffer.
The anti-IL4R$\alpha$ antibody in the present disclosure or control was serially 4-fold diluted from 80 nM in a PBST buffer containing 2.5% w/v of skim milk, added at 100 $\mu$L/well to a plate coated with IL4R$\alpha$, and co-incubated with human IL4R$\alpha$-his protein for 40 min at 37 °C. The plate was washed 4 times with the washing buffer before 0.56 nM of biotinylated human IL4 protein (Sino Biological Inc., Cat. No. 11846-HNAE) was added at 100 $\mu$L per well. The plate was then incubated for 40 min at 37 °C. The plate was washed again with the washing buffer before an HRP-labeled streptavidin (1:10000 diluted in a PBST buffer, Jackson Immunoresearch, Cat. No. 016-030-084) was added at 100 $\mu$L/well. The plate was then incubated for 40 min at 37 °C. The plate was washed again with the washing buffer. Finally, TMB was added and the reaction was stopped by adding 1 M H$_2$SO$_4$. The absorbance at 450 nm was measured. Data were analyzed using Graphpad Prism software and IC$_{50}$ values were reported.

*3.2 Benchmark blocking ELISA*

The capacity of the anti-IL4R$\alpha$ antibodies in the present disclosure to block the benchmark-human IL4R$\alpha$ binding was determined by competitive ELISA. Briefly, the benchmark in PBS at 2 $\mu$g/mL was immobilized on a 96-well plate at 100 $\mu$L/well and incubated overnight at 4 °C. The next day, the plate was washed with a washing buffer (PBS + 0.05% w/v Tween-20, PBST) before a PBST containing 5% w/v of skim milk was added. The plate was then incubated at 37 °C for 2 h for blocking. During the blocking of the 96-well plate, the anti-IL4Ra antibodies of the present disclosure or control was serially 4-fold diluted from 100 nM with a biotinylated human IL4R$\alpha$-his protein (prepared in-house, amino acid sequence set forth in SEQ ID NO: 15, dissolved in a PBST containing 2.5% w/v of skim milk at a concentration of 0.55 nM) and incubated at 25 °C for 40 min. After the plate was washed, the antibody/IL4R$\alpha$-his mixture was added at 100 $\mu$L/well to the plate coated with the benchmark. The plate was incubated at 37 °C for 40 min and washed with the washing buffer. An HRP-labeled streptavidin was added at 100 $\mu$L/well, and the plate was incubated at 37 °C for 40 min to detect biotinylated human IL4R$\alpha$-his bound to the plate. The plate was washed again with the washing buffer. Finally, TMB was added and the reaction was stopped by adding 1 M H$_2$SO$_4$. The absorbance at 450 nm was measured. Data were analyzed using Graphpad Prism software and IC$_{50}$ values were reported.

*3.3 Cell-based ligand blocking FACS*

The blocking effect of anti-IL4R$\alpha$ antibodies on the binding activity of IL4 protein to cell surface IL4R$\alpha$ was assessed by flow cytometry (FACS) using the 293F-IL4R$\alpha$ cells prepared above. Briefly, the 293F-IL4R$\alpha$ cells were harvested from cell culture flasks, washed twice, and resuspended in an FACS buffer (a PBS containing 2% v/v of fetal bovine serum). Then, the anti-IL4R$\alpha$ antibodies or control serially 4-fold diluted in the FACS buffer from 80 nM was added at 100 $\mu$L/well to a 96-well plate containing $1 \times 10^5$ cells/well. The plate was incubated in an ice bath for 40 min. The plate was washed twice with the FACS buffer before 1.67 nM of biotinylated human IL4 protein (Sino Biological Inc., Cat. No. 11846-HNAE) was added at 100 $\mu$L perwell. The plate was then incubated at 4 °C for 40 min in the dark. The plate was washed twice with the FACS buffer before an R-phycoerythrin-labeled streptavidin (1:500 diluted in the FACS buffer, Jackson Immunoresearch, Cat. No. 016-110-084) was added at 100 $\mu$L/well. The plate was then incubated at 4 °C for 40 min in the dark. The cells were washed twice and then resuspended in the FACS buffer. Fluorescence values were measured using Becton Dickinson FACS Canto II-HTS. Data were analyzed using Graphpad Prism software and IC$_{50}$ values were reported.

**Example 4** Cell-based function assay of anti-IL4R$\alpha$ antibodies

IL4 and IL13 can bind to IL4R$\alpha$ and induce STAT6 phosphorylation in HEK293T-IL4R$\alpha$-STAT6-STAT6LUC-LB2 cells. STAT6 phosphorylation is crucial in the IL4/IL13 signaling pathway.

Briefly, HEK293T cells (ATCC CRL-11268) naturally expressing IL13R$\alpha$1 were stably transfected with pcDNA3.1-Puro (YouBio Biological Inc., Cat. No. VT9222) plasmid constructs (having nucleotides encoding human IL4R$\alpha$ between BamHI and XhoI), STAT6 plasmid (Sino Biological Inc., Cat. No. HG13190-NH; having nucleotides encoding human STAT6 between KpnI and XbaI), and STAT6 luciferase reporter gene plasmid STAT6-Luc (Yeasen Biological Inc., Cat. No. 11588ES03) to prepare the HEK293T-IL4R$\alpha$-STAT6-STAT6LUC-LB2 cells in-house. Single-cell clone LB2 were then selected for all the subsequent function assays.

The inhibitory effect of anti-IL4R$\alpha$ antibodies in the present disclosure on STAT6 phosphorylation induced by IL4 and IL13 was examined.

Briefly, HEK293T-IL4R$\alpha$-STAT6-STAT6LUC-LB2 cells at log phase were resuspended in a culture medium (RPMI1640 + 10% FBS) and seeded on a 96-well plate at 100 $\mu$L/well, each well containing $5 \times 10^5$ cells. The anti-IL4R$\alpha$ antibodies in the present disclosure or controls (including an anti-CD22 antibody prepared in-house) serially 5-fold diluted from 100 nM were added at 50 $\mu$L per well. The plate was incubated for 30 min at 37 °C. IL4 protein (600 pg/mL, Sino Biological Inc., Cat. No. 11846-HNAE) or IL13 protein (50 ng/mL, Sino Biological Inc., Cat. No. 10369-HNAC) was added at 50 $\mu$L per well and incubated at 37 °C for 20 min. The plate was centrifuged and washed twice with a staining buffer (prepared in-house, DPBS + 0.5% w/v BSA + 2 mM EDTA) before a fixation buffer (BD Biosciences Inc., Cat. No. 5545655) was added at 50 $\mu$L per well. The plate was then incubated at 4 °C for 30 min. The cells were washed twice before a permeabilization buffer (BD Biosciences Inc., Cat. No. 558050) was added at 200 $\mu$L per well. The cells were then incubated for 30 min in an ice bath. The plate was washed thrice with the staining buffer. An anti-pSTAT6 antibody (20-fold diluted from a pSTAT6 stock solution, BD Biosciences Inc., Cat. No. 562079) was then added, and the plate was stood on ice for 60 min. The plates were finally washed twice before the cells were resuspended in the staining buffer. The fluorescence values were measured using Becton Dickinson FACS Canto II-HTS. Data were analyzed using Graphpad Prism softwareand IC$_{50}$ values were reported.

**Example 5** Production and characterization of chimeric antibody

The heavy chain variable region and the light chain variable region of a mouse anti-IL4R$\alpha$ monoclonal antibody were sequenced and the sequence IDs are summarized in Table 1.

The heavy chain variable region and the light chain variable region of the mouse anti-IL4R$\alpha$ monoclonal antibody C2C1A1A1 were cloned into a vector containing the human IgG4 heavy chain constant region (SEQ ID NO: 13) and a vector containing the human $\kappa$ light chain constant region (SEQ ID NO: 14), respectively, wherein the C terminus of the variable regions were linked to the N terminus of the respective constant regions.

The vector containing the nucleotides encoding the heavy chain variable region linked to the human IgG4 heavy chain constant region and the vector containing the nucleotides encoding the light chain variable region linked to the human $\kappa$ light chain constant region were transiently transfected into 50 mL of 293F cell suspension with 1 mg/mL PEI in a light chain construct heavy chain construct ratio of 60%:40%.

After six days of culture in shake flasks, the cell supernatant was harvested, and the cells in the supernatant were collected by centrifugation and filtered through a 0.22 $\mu$m filter for immunoglobulin isolation. The chimeric antibody was purified by protein A affinity chromatography. Briefly, a protein A sepharose column (Bestchrom (Shanghai) Biosciences, Cat. No. AA0273) was washed with 5 to 10 column volumes of a PBS buffer. The cell supernatant was loaded on the protein A sepharose column before the column was washed with the PBS buffer until the protein absorbance reached the baseline. The column was washed with an elution buffer (0.1 M glycine-HCl, pH 2.7) and the eluate was immediately collected in 1.5-mL tubes and neutralized with a neutralization buffer (1 M Tris-HCl, pH 9.0). Fractions containing the immunoglobulin were pooled and dialyzed overnight at 4 °C in PBS. The purified antibodies were measured by capture ELISA, competitive ELISA, BIAcore affinity assay, cell-based binding FACS assay, and cell-based function assay as per the protocols in the examples above. For mouse C2C1A1A1, the detection protocols were modified on the basis of the previous example as described below:

For capture ELISA, the goat anti-human IgG was replaced by a 2 $\mu$g/mL goat anti-mouse IgG (Fc$\gamma$ fragment-specific, Jackson Immunoresearch, Cat. No. 115-005-008), 100 $\mu$L/well;

For BIAcore, the goat anti-human IgG was replaced by a goat anti-mouse IgG (GE Healthcare, Cat. No. BR100838, Mouse Antibody Capture Kit) covalently linked to a CM5 chip;

For cell-based binding FACS, the R-phycoerythrin affinipure goat anti-human IgG was replaced by an R-phycoerythrin affinipure F(ab')$_2$ fragment goat anti-mouse IgG (Jackson Immunoresearch, Cat. No. 115-116-146), 100 μL/well.

The results are shown in Table 2, and the data indicate that the chimeric antibody has similar binding affinity/capacity and blocking activity to its parent mouse antibody.

Table 2. Binding and function activity of chimeric antibody

| mAb ID# | Capture ELISA for human IL4R ($EC_{50}$, nM) | BIAcore affinity to human IL4R($K_D$, M) | Cell-based binding FACS ($EC_{50}$, nM) | IL4-IL4Rα blocking ELISA ($IC_{50}$, nM) i | Cell-based function assay | |
|---|---|---|---|---|---|---|
| | | | | | Inhibition of IL4-induced STAT6 phosphorylat on ($IC_{50}$, nM) | Inhibition of IL13-induced STAT6 phosphorylati on ($IC_{50}$, nM) |
| Mouse C2C1A1A1 | 0.03 | 1.195E-10 | 0.31 | 1.89 | 0.19 | 0.27 |
| Chimeric C2C1A1A1 | 0.05 | 5.219E-10 | 0.63 | 3.12 | 0.27 | 0.56 |
| Benchmark | 0.05 | 4.656E-10 | 0.46 | 2.06 | 0.37 | 0.48 |

**Example 6** Humanization of anti-IL4Rα monoclonal antibody

The mouse anti-IL4Rα antibody C2C1A1A1 was humanized and further characterized. The humanization of the mouse antibody was conducted using an established CDR grafting method, as described below.

To select acceptor framework for the humanization of the mouse antibody C2C1A1A1, the sequences of light and heavy chain variable regions of each mouse antibody were blasted against the human immunoglobulin gene database. The human germline antibody with the highest homology was selected as the acceptor framework for humanization. The CDRs of the heavy/light chain variable region of the mouse antibody were inserted into the selected framework and the residues in the framework regions were further backmutated to obtain more candidate heavy/light chain variable regions. A total of 16 exemplary humanized C2C1A1A1 antibodies were obtained and denoted as huC2C1A1A1-V1 to huC2C1A1A1-V16. The heavy/light chain variable region sequence IDs are shown in Table 1.

The vector containing the nucleotides encoding the humanized heavy chain variable region linked to the human IgG4 heavy chain constant region (SEQ ID NO: 13) and the vector containing the nucleotides encoding the humanized light chain variable region linked to the human κ light chain constant region (SEQ ID NO: 14) were transiently transfected into 50 mL of 293F cell suspension with 1 mg/mL PEI in a light chain construct heavy chain construct ratio of 60%:40%.

After six days of culture in shake flasks, the cell supernatant was harvested, and the cells in the supernatant were collected by centrifugation and filtered through a 0.22 μm filter for immunoglobulin isolation. The antibody was purified by protein A affinity chromatography. Briefly, a protein A sepharose column (Bestchrom (Shanghai) Biosciences, Cat. No. AA0273) was washed with 5 to 10 column volumes of a PBS buffer. The cell supernatant was loaded on the protein A sepharose column before the column was washed with the PBS buffer until the protein absorbance reached the baseline. The column was washed with an elution buffer (0.1 M glycine-HCl, pH 2.7) and the eluate was immediately collected in 1.5-mL tubes and neutralized with a neutralization buffer (1 M Tris-HCl, pH 9.0). Fractions containing the immunoglobulin were pooled and dialyzed overnight at 4 °C in PBS.

**Example 7** Characterization of humanized antibodies

According to the protocol in the examples above, the binding affinity of the humanized antibodies to human IL4Rα was assessed by the BIAcore technique, and the $K_a$, $K_d$, and $K_D$ values were determined and summarized in Table 3.

Table 3. Binding affinity of humanized C2C1A1A1 monoclonal antibodies

| mAb | BIAcore kinetics | | | | | |
|---|---|---|---|---|---|---|
| | Human IL4Rα | | | | | |
| | Ka (1/Ms) | Kd (1/s) | $K_D$ (M) | Start (RU) | End (RU) | Dissociation (%) |
| Chimeric C2C1A1A1 | 3.29E+05 | <1.00E-05 | <3.04E-11 | 67.1 | 65.8 | -1.94% |
| Supernatant containing chimeric C2C1A1A1 | 3.64E+05 | 4.04E-05 | 1.11E-10 | 40 | 40.4 | 1.00% |
| huC2C1A1A1-V1 | 1.29E+05 | <1.00E-05 | <7.73E-11 | 43.9 | 44 | 0.23% |
| huC2C1A1A1-V2 | 2.26E+05 | <1.00E-05 | <4.42E-11 | 50.4 | 50.3 | -0.20% |
| huC2C1A1A1-V3 | 2.57E+05 | <1.00E-05 | <3.89E-11 | 48.6 | 48.3 | -0.62% |
| huC2C1A1A1-V4 | 4.55E+05 | <1.00E-05 | <2.20E-11 | 49.5 | 49.6 | 0.20% |
| huC2C1A1A1-V5 | 2.65E+06 | 7.50E-05 | 2.83E-11 | 65.8 | 64.3 | -2.28% |
| huC2C1A1A1-V6 | 3.83E+05 | <1.00E-05 | <2.61E-11 | 50.9 | 50.6 | -0.59% |
| huC2C1A1A1-V7 | 2.63E+05 | <1.00E-05 | <3.80E-11 | 60.5 | 60.1 | -0.66% |
| huC2C1A1A1-V8 | 2.78E+05 | <1.00E-05 | <3.60E-11 | 65.2 | 64.4 | -1.23% |
| huC2C1A1A1-V9 | 1.97E+05 | <1.00E-05 | <5.07E-11 | 41.2 | 41.1 | -0.24% |
| huC2C1A1A1-V10 | 3.29E+05 | <1.00E-05 | <3.04E-11 | 64.9 | 63.9 | -1.54% |
| huC2C1A1A1-V11 | 3.30E+05 | <1.00E-05 | <3.03E-11 | 54.2 | 53.7 | -0.92% |
| huC2C1A1A1-V12 | 3.02E+05 | 1.42E-05 | 4.70E-11 | 53.1 | 52.1 | -1.88% |
| huC2C1A1A1-V13 | 1.193E+05 | <1.00E-05 | <8.38E-11 | 36.4 | 36.7 | 0.82% |
| huC2C1A1A1-V14 | 1.53E+05 | <1.00E-05 | <6.54E-11 | 50.1 | 50.5 | 0.80% |
| huC2C1A1A1-V15 | 2.30E+05 | <1.00E-05 | <4.35E-11 | 69 | 69.2 | 0.29% |
| huC2C1A1A1-V16 | 2.96E+05 | <1.00E-05 | <3.38E-11 | 47.3 | 47.1 | -0.42% |
| Benchmark | 3.33E+05 | 6.25E-05 | 1.88E-10 | 71.9 | 70 | -2.64% |

$$\text{Dissociation (\%)} = (\text{End (RU)} - \text{Start (RU)})/\text{Start (RU)}$$

The results suggest that the humanized antibodies have similar binding affinity for human IL4Rα to the chimeric antibody, and compared to the benchmark, all the humanized huC2C1A1A1 antibodies exhibited higher binding affinity for human IL4Rα.

According to the protocol in the examples above, the humanized antibodies huC2C1A1A1-V14 and huC2C1A1A1-V15 were further analyzed by Biacore, capture ELISA, indirect ELISA, cell-based binding FACS, competitive ELISA, cell-based ligand blocking FACS, and cell-based function assay.

The thermal stability of the humanized antibody huC2C1A1A1-V15 was also examined. Briefly, Tm (melting temperature) was determined by protein thermal shift assay using the GloMelt™ Thermal Shift Protein Stability Kit (Biotium, Cat. No. 33022-T, Lot No. 181214). Briefly, the GloMelt™ dye was thawed to room temperature. The vial containing the dye was vortexed and centrifuged. Then, 5 μL of a 200× dye was added to 95 μL of PBS to prepare a 10× dye. 2 μL of the 10× dye and 10 μg of the humanized antibody were added to the reaction system, and PBS was added to bring the total reaction volume to 20 μL. The centrifuge tubes containing the dye and the antibodies were centrifuged briefly and placed in a real-time PCR thermal cycler (Roche, LightCycler 480 II) in which the Melt Curve program was set according to the parameters in Table 4. The results are shown in Tables 5-1, 5-2, and 5-3, and the data demonstrate that the humanized C2C1A1A1 antibodies exhibited comparable, if not better, binding affinity/activity for human IL4Rα and blocking capacity for IL4Rα-IL4/IL13 as compared to the benchmark.

Table 4. Parameters of Melt Curve program

| Profile procedures | Temperature | Ramp rate | Duration |
|---|---|---|---|
| Initial hold | 25 °C | NA | 30 s |
| Melt curve | 25-99 °C | 0.1 °C/s | NA |

Table 5-1. Binding activity and functional activity of humanized monoclonal antibodies

| mAb ID# | Binding assay | | | | |
|---|---|---|---|---|---|
| | Human IL4Rα-his | | | Cynomolgus monkey IL4Rα-his | |
| | Capture ELISA (EC$_{50}$, nM) | Biacore (K$_D$, M) | Cell-based binding FACS (EC$_{50}$, nM) | Biacore (K$_D$, M) | Indirect ELISA (EC$_{50}$, nM) |
| Chimeric C2C1A1A1 | 0.05 | 3.88E-10 | 0.33 | Weak binding | 0.60 |
| huC2C1A1A1-V15 | 0.05 | 4.47E-10 | 0.31 | Weak binding | 0.52 |
| huC2C1A1A1-V14 | 0.06 | 6.42E-10 | 0.39 | Weak binding | 0.68 |
| Benchmark | 0.05 | 7.46E-10 | 0.40 | No binding | 0.67 |

Table 5-2. Binding activity and functional activity of humanized monoclonal antibodies

| mAb ID# | Binding assay | | Competitive ELISA | | |
|---|---|---|---|---|---|
| | Marmoset monkey IL4Rα-his | | IL4-IL4Rα blocking ELISA (IC$_{50}$, nM) | Benchmark blocking ELISA (IC$_{50}$, nM) | Cell-based ligand blocking FACS (IC$_{50}$, nM) |
| | Biacore (K$_D$, M) | Indirect ELISA (EC$_{50}$, nM) | | | |
| Chimeric C2C1A1A1 | Weak binding | 0.62 | 2.46 | 0.17 | 0.48 |
| huC2C1A1A1-V15 | Weak binding | 0.41 | 2.03 | 0.16 | 0.50 |
| huC2C1A1A1-V14 | Weak binding | 0.55 | 2.17 | 0.17 | 0.58 |
| Benchmark | No binding | 1.72 | 1.74 | 0.19 | 0.42 |

Table 5-3. Binding activity and functional activity of humanized monoclonal antibodies

| mAb ID# | Cell-based function assay (IC$_{50}$, nM) | | Tm (melting temperature, °C) | |
|---|---|---|---|---|
| | Inhibition of IL4-induced STAT6 phosphorylation | Inhibition of IL13-induced STAT6 phosphorylation | Tm1 | Tm2 |
| Chimeric C2C1A1A1 | 0.26 | 0.39 | * | * |
| huC2C1A1A1-V15 | 0.61 | 0.60 | NA | 72.0 |
| huC2C1A1A1-V14 | 0.64 | 0.73 | 65.5 | 74.0 |
| Benchmark | 0.70 | 0.84 | * | * |

Note: "*" means not tested yet.

**Example 8** Preparation and screening of pharmaceutical compositions

Detection method:

(1) Size exclusion chromatography (SEC-UPLC): for determining the purity of the antibodies in the sample; Thermo Vanquish F high-performance liquid chromatography was used, with Waters ACQUITY UPLC Protein BEH SEC Column (200 Å, 1.7 μm, 4.6 mm × 300 mm) as the column and Waters ACQUITY UPLC Protein BEH SEC Guard Column (200 Å, 1.7 μm, 4.6 mm × 30 mm) as the pre-column; the elution was conducted using 50 mmol/L phosphate buffer + 200 mmol/L sodium chloride solution (pH 7.0) as the mobile phase, and the detection wavelength was 280 nm. The percentage contents of the macromolecular impurities and the immunoglobulin monomer were calculated by area normalization.

(2) Differential scanning fluorimetry (DSF): the unfolding temperature (Tm) of the samples was analyzed on a protein stability analyzer (NanoTemper, Prometheus NT.48); 10 μL of the sample was transferred into the sample chamber by a capillary, and measured by the following program: the start temperature of scanning was 25 °C, the end temperature of scanning was 95 °C, and the temperature ramping rate was 0.3 °C/min.

(3) The samples were analyzed for viscosity on an HVROC-S viscometer (Rheosense, μVISC) with the auto mode, and the analysis results were recorded.

(4) Whole column imaging capillary isoelectric focusing (iCIEF): for determining the charge variants of the sample; an imaging capillary isoelectric focusing electrophoresis apparatus (ProteinSimple iCE3), a rapid automatic protein characterization system (Protein Simple, Maurice), a coated silica capillary (Protein Simple), and an ultraviolet detector were adopted for detection at the ultraviolet wavelength 280 nm. The sample injection duration was set to 60 seconds, the pre-focusing duration was set to 1 minute at 1500 V, and the focusing duration was set to 10 minutes at 300 V. The percentage peak area of the charge variants was calculated by peak area normalization.

Preparation of buffering agents:

(1) The histidine-histidine hydrochloride buffering agent comprises histidine and histidine hydrochloride; in a specific example, the histidine-histidine hydrochloride buffering agent comprises histidine and histidine hydrochloride monohydrate; for example, a 20 mM histidine-histidine hydrochloride buffering agent (pH 5.8) consists of about 1.17 mg/mL of histidine and about 2.62 mg/mL of histidine hydrochloride monohydrate;

(2) The sodium phosphate buffering agent comprises disodium hydrogen phosphate and sodium dihydrogen phosphate; in a specific example, the sodium phosphate buffering agent comprises sodium dihydrogen phosphate monohydrate and disodium hydrogen phosphate dodecahydrate; for example, a 20 mM sodium phosphate buffering agent (pH 6.0) is formulated from about 2.539 mg/mL of sodium dihydrogen phosphate monohydrate and about 0.573 mg/mL of disodium hydrogen phosphate dodecahydrate;

(3) The sodium acetate-acetic acid buffering agent comprises sodium acetate and acetic acid; in a specific example, the sodium acetate-acetic acid buffering agent comprises sodium acetate trihydrate and acetic acid; for example, a 20 mM sodium acetate-acetic acid buffering agent (pH 6.0) is formulated from about 2.605 mg/mL of sodium acetate trihydrate and about 0.141 mg/mL of acetic acid.

The anti-IL4Rα antibody (huC2C1A1A1-V15) was transferred by ultrafiltration into the buffering agents in Table 6, and concentrated. Then the surfactants and stabilizers were added according to Table 6. After mixing, the mixture was filtered through a 0.22 μm filter membrane for sterilization, and aliquoted into vials. The vials were stoppered and capped. The SEC-UPLC results of pharmaceutical compositions F1-F4 are shown in Table 7, wherein the increase in macromolecular impurities of F4 (from 1.36% to 5.66%) was higher than those of F1-F3 after 1 month of storage at 40 °C.

Table 6. Components of pharmaceutical compositions

| No. | Anti-IL4Rα antibody | Buffering agent | Surfactant | Stabilizer |
|---|---|---|---|---|
| F1 | 175 mg/mL | 20 mM histidine-histidine hydrochloride, pH 5.0 | 0.4 mg/mL polysorbate 80 | 50 mg/mL sucrose |
| F2 | 175 mg/mL | 20 mM histidine-histidine hydrochloride, pH 6.0 | 0.4 mg/mL polysorbate 80 | 50 mg/mL sucrose |
| F3 | 175 mg/mL | 20 mM sodium phosphate, pH 6.0 | 0.4 mg/mL polysorbate 80 | 50 mg/mL sucrose |
| F4 | 175 mg/mL | 20 mM sodium acetate-acetic acid, pH 6.0 | 0.4 mg/mL polysorbate 80 | 50 mg/mL sucrose |

Table 7. SEC-UPLC results of pharmaceutical compositions

| Sample | Sampling points | SEC-UPLC (%) | |
|---|---|---|---|
| | | Macromolecular impurities | Main peak |
| F1 | Day 0 | 0.77 | 99.02 |
| | 40 °C, week 1 | 1.29 | 97.37 |
| | 40 °C, week 2 | 1.57 | 96.26 |
| | 40 °C, month 1 | 2.46 | 93.68 |
| F2 | Day 0 | 0.95 | 98.84 |
| | 40 °C, week 1 | 1.5 | 98.27 |
| | 40 °C, week 2 | 1.9 | 97.44 |
| | 40 °C, month 1 | 2.42 | 96.83 |
| F3 | Day 0 | 1.35 | 98.44 |
| | 40 °C, week 1 | 2.16 | 96.93 |
| | 40 °C, week 2 | 2.44 | 96.54 |
| | 40 °C, month 1 | 3.37 | 95.46 |
| F4 | Day 0 | 1.36 | 98.44 |
| | 40 °C, week 1 | 2.43 | 97.34 |
| | 40 °C, week 2 | 2.10 | 96.35 |
| | 40 °C, month 1 | 5.66 | 93.45 |

Illustratively, 20 mM histidine-histidine hydrochloride was selected as the buffering agent. The anti-IL4Rα antibody (huC2C1A1A1-V15) was transferred by ultrafiltration into the buffering agents in Table 8, and concentrated. Then the surfactants and stabilizers were added according to Table 8. After mixing, the mixture was filtered through a 0.22 $\mu$m filter membrane for sterilization, and aliquoted into vials. The vials were stoppered and capped. The Tm and SEC-UPLC results of pharmaceutical compositions F5-F7 are shown in Table 9, wherein the increase in macromolecular impurities of F6 was the least after 1 month of storage at 40 °C.

Table 8. Components of pharmaceutical compositions

| No. | Anti-IL4Rα antibody | Buffering agent | Surfactant | Stabilizer | | |
|---|---|---|---|---|---|---|
| F5 | 150 mg/mL | 20 mM histidine-histidine hydrochloride, pH 5.8 | 0.4 mg/mL polysorbate 80 | 50 mg/mL sucrose | 50 mM arginine hydrochloride | / |
| F6 | 150 mg/mL | 20 mM histidine-histidine hydrochloride, pH 5.8 | 0.4 mg/mL polysorbate 80 | / | 250 mM proline | 20 mM sodium chlorid e |
| F7 | 150 mg/mL | 20 mM histidine-histidine hydrochloride, pH 5.8 | 0.4 mg/mL polysorbate 80 | 50 mg/mL sucrose | 250 mM proline | 20 mM sodium chlorid e |
| "/" denotes none. | | | | | | |

Table 9. Tm and SEC-UPLC results of pharmaceutical compositions

| Sample | Sampling points | Tm (°C) | | SEC-UPLC (%) | |
|---|---|---|---|---|---|
| | | Tm Onset | Tm1 | Macromolecular impurities | Main peak |
| F5 | Day 0 | 60.0 | 64.4 | 0.79 | 98.28 |
| | 40 °C, week 1 | * | * | 1.12 | 98.04 |
| | 40 °C, week 2 | * | * | 1.34 | 97.68 |
| | 40 °C, month 1 | * | * | 1.59 | 97.27 |
| | 2-8 °C, month 1 | * | * | 0.83 | 98.3 |
| F6 | Day 0 | 60.0 | 64.3 | 0.81 | 98.35 |
| | 40 °C, week 1 | * | * | 1.25 | 97.82 |
| | 40 °C, week 2 | * | * | 1.46 | 97.52 |
| | 40 °C, month 1 | * | * | 1.42 | 97.44 |
| | 2-8 °C, month 1 | * | * | 0.86 | 98.32 |
| F7 | Day 0 | 60.6 | 65.2 | 0.76 | 98.52 |
| | 40 °C, week 1 | * | * | 1.19 | 97.88 |
| | 40 °C, week 2 | * | * | 1.42 | 97.58 |
| | 40 °C, month 1 | * | * | 1.74 | 97.2 |
| | 2-8 °C, month 1 | * | * | 0.86 | 98.35 |
| Note: "*" means not tested yet. | | | | | |

Illustratively, 20 mM histidine-histidine hydrochloride was selected as the buffering agent. The anti-IL4Rα antibody (huC2C1A1A1-V15) was transferred by ultrafiltration into the buffering agents in Table 10, and concentrated. Then the surfactants and stabilizers were added according to Table 10. After mixing, the mixture was filtered through a 0.22 μm filter membrane for sterilization, and aliquoted into vials. The vials were stoppered and capped. The Tm, viscosity, and SEC-UPLC and iCIEF results of pharmaceutical compositions F8 and F9 are shown in Table 11, where F8 demonstrated a lower viscosity than F9.

Table 10. Components of pharmaceutical compositions

| No. | Anti-IL4Rα antibody | Buffering agent | Surfactant | Stabilizer | |
|---|---|---|---|---|---|
| F8 | 150 mg/mL | 20 mM histidine-histidine hydrochloride, pH 5.8 | 0.4 mg/mL polysorbate 80 | 250 mM proline | 20 mM sodium chloride |
| F9 | 150 mg/mL | 20 mM histidine-histidine hydrochloride, pH 5.8 | 0.4 mg/mL polysorbate 80 | 350 mM proline | / |
| "/" denotes none. | | | | | |

Table 11. Tm, viscosity, and SEC-UPLC and iCIEF results of pharmaceutical compositions

| Sample | Sampling points | Tm (°C) | | Viscosity (mPa.s) | SEC-UPLC (%) | | iCIEF (%) | | |
|---|---|---|---|---|---|---|---|---|---|
| | | Tm Onset | Tm1 | | Macromolecular impurities | Main peak | Acidic variants | Main peak | Basic variants |
| F8 | Day 0 | 59.4 | 63.8 | 12.46 | 0.89 | 98.76 | 16.0 | 74.6 | 9.4 |
| | 40 °C, week 1 | * | * | * | 1.29 | 98.27 | 19.3 | 70.0 | 10.7 |
| | 40 °C, month 1 | * | * | * | 1.67 | 97.17 | 31.6 | 59.9 | 8.6 |
| | 2-8 °C, week 1 | * | * | * | 0.95 | 98.73 | / | / | / |
| | 2-8 °C, month 1 | * | * | * | 0.98 | 98.67 | 18.8 | 71.0 | 10.2 |
| | 2-8 °C, month 3 | * | * | * | 0.91 | 98.7 | 19.9 | 71.3 | 8.8 |
| | 2-8 °C, month 6 | * | * | * | 1.07 | 98.58 | 20.7 | 69.1 | 10.2 |
| F9 | Day 0 | 60.4 | 64.7 | 15.98 | 0.77 | 98.99 | 15.7 | 74.9 | 9.4 |
| | 40 °C, week 1 | * | * | * | 1.24 | 98.4 | 19.1 | 70.8 | 10.1 |
| | 40 °C, month 1 | * | * | * | 1.47 | 97.78 | 33.4 | 59.0 | 7.6 |
| | 2-8 °C, week 1 | * | * | * | 1.00 | 98.66 | / | / | / |
| | 2-8 °C, month 1 | * | * | * | 0.96 | 98.72 | 20.4 | 70.6 | 8.9 |
| | 2-8 °C, month 3 | * | * | * | 0.92 | 98.7 | 19.5 | 69.7 | 10.8 |
| | 2-8 °C, month 6 | * | * | * | 1.08 | 98.44 | 20.1 | 70.7 | 9.2 |

Note: "*"means not tested yet.

Although the present disclosure has been described in conjunction with one or more embodiments, it will be appreciated that the present disclosure is not limited to such embodiments, but is intended to encompass all alternatives, modifications, and equivalents included within the spirit and scope of the appended claims. All the references cited herein are incorporated by reference in their entireties.

The sequence information of the present disclosure is summarized in Table 12 below.

Table 12. Sequence information

| Description Sequence/SEQ ID NO. |
|---|
| VH-CDR1 of mouse, chimeric, and humanized C2C1A1A1 TYGMS (SEQ ID NO: 1) |
| VH-CDR2 of mouse, chimeric, and humanized C2C1A1A1 TINSNGGSTSYPDSVKG (SEQ ID NO: 2) |
| VH-CDR3 of mouse, chimeric, and humanized C2C1A1A1 |

(continued)

| Description Sequence/SEQ ID NO. |
| --- |
| FFRFRNAMDY (SEQ ID NO: 3) |
| VL-CDR1 of mouse, chimeric, and humanized C2C1A1A1 RTSENIYSYLA (SEQ ID NO: 4) |
| VL-CDR2 of mouse, chimeric, and humanized C2C1A1A1 NAKTLAE (SEQ ID NO: 5) |
| VL-CDR3 of mouse, chimeric, and humanized C2C1A1A1 QHYYGPPTWT (SEQ ID NO: 6) |
| VH of mouse and chimeric C2C1A1A1 |
| EVQLVESGGGLVQPGGSLKLSCAASGFTFSTYGMSWVRQTPDKRLELVATINSNGGST SYPDSVKGRFTISRDNAKNTLYLQMSSLKSEDTAMFYCARFFRFRNAMDYWGQGTSVT VSS (SEQ ID NO: 7) |
| VH of mouse C2C1A1A1 |
| GAGGTGCAGCTGGTGGAGTCTGGGGGAGGCTTAGTGCAGCCTGGAGGGTCCCTGA AACTCTCCTGTGCAGCCTCTGGATTCACTTTCAGTACTTATGGCATGTCTTGGGTTCG CCAGACTCCAGACAAGAGGCTGGAGTTGGTCGCAACCATTAATAGTAATGGTGGTAG TACCAGTTATCCAGACAGTGTGAAGGGCCGATTCACCATCTCCAGAGACAATGCCAA GAACACCCTGTACCTGCAAATGAGCAGTCTGAAGTCTGAGGACACAGCCATGTTTTA CTGTGCAAGATTTTTCCGCTTTAGGAATGCTATGGACTACTGGGGTCAAGGAACCTC AGTCACCGTCTCCTCA (SEQ ID NO: 17) |
| VH of chimeric C2C1A1A1 |
| GAGGTGCAGCTGGTGGAGAGCGGCGGCGGACTGGTGCAGCCTGGAGGATCCCTGA AGCTGTCCTGCGCCGCCTCCGGCTTCACCTTCTCCACATACGGCATGTCCTGGGTGA GACAGACCCCTGATAAGAGACTGGAGCTGGTGGCCACCATCAACAGCAACGGCGGC AGCACCAGCTACCCCGACAGCGTGAAGGGCAGATTCACCATCTCCAGAGACAACGC CAAGAACACCCTGTACCTGCAGATGTCCAGCCTGAAGAGCGAGGATACAGCCATGTT CTACTGTGCCAGGTTCTTTAGGTTCAGAAATGCCATGGACTACTGGGGCCAGGGCAC CTCCGTGACAGTGAGCAGC (SEQ ID NO: 18) |
| VH of huC2C1A1A1-V1 to huC2C1A1A1-V4 |
| EVQLVESGGGLVQPGGSLRLSCAASGFTFSTYGMSWVRQAPGKGLVX$_1$VX$_2$TINSNGGS TSYPDSVKGRFTISRDNAKNTLYLQMNSLRAEDTAVYYCARFFRFRNAMDYWGQGTLVT VSS (SEQ ID NO: 8) X$_1$ = W, X$_2$ = S EVQLVESGGGLVQPGGSLRLSCAASGFTFSTYGMSWVRQAPGKGLVWVSTINSNGGST SYPDSVKGRFTISRDNAKNTLYLQMNSLRAEDTAVYYCARFFRFRNAMDYWGQGTLVT VSS |
| VH of huC2C1A1A1-V5 to huC2C1A1A1-V8 |

(continued)

| Description Sequence/SEQ ID NO. |
|---|
| EVQLVESGGGLVQPGGSLRLSCAASGFTFSTYGMSWVRQSPDKRLEWVSTINSNGGST SYPDSVKGRFTISRDNAKNTLYLQMRSLRAEDTAVYYCARFFRFRNAMDYWGQGTLVT VSS (SEQ ID NO: 9) |
| VH of huC2C1A1A1-V9 to huC2C1A1A1-V12 |
| EVQLVESGGGLVQPGGSLRLSCAASGFTFSTYGMSWVRQAPGKGLVX$_1$VX$_2$TINSNGGS TSYPDSVKGRFTISRDNAKNTLYLQMNSLRAEDTAVYYCARFFRFRNAMDYWGQGTLVT VSS (SEQ ID NO: 8) X$_1$ = L, X$_2$ = A |
| EVQLVESGGGLVQPGGSLRLSCAASGFTFSTYGMSWVRQAPGKGLVLVATINSNGGST SYPDSVKGRFTISRDNAKNTLYLQMNSLRAEDTAVYYCARFFRFRNAMDYWGQGTLVT VSS |
| VH of huC2C1A1A1-V13 to huC2C1A1A1-V16 |
| EVQLVESGGGLVQPGGSLRLSCAASGFTFSTYGMSWVRQAPGKGLVX$_1$VX$_2$TINSNGGS TSYPDSVKGRFTISRDNAKNTLYLQMNSLRAEDTAVYYCARFFRFRNAMDYWGQGTLVT VSS (SEQ ID NO: 8) X$_1$ = W, X$_2$ = A |
| EVQLVESGGGLVQPGGSLRLSCAASGFTFSTYGMSWVRQAPGKGLVWVATINSNGGST SYPDSVKGRFTISRDNAKNTLYLQMNSLRAEDTAVYYCARFFRFRNAMDYWGQGTLVT VSS GAGGTGCAGCTGGTGGAGTCCGGAGGAGGACTGGTGCAGCCCGGCGGCTCTCTGA GACTGAGCTGCGCTGCCTCCGGCTTCACCTTTAGCACCTACGGCATGAGCTGGGTG AGACAAGCCCCCGGCAAAGGACTGGTGTGGGTGGCTACCATCAACAGCAACGGCG GCTCCACAAGCTACCCCGACAGCGTGAAGGGAAGATTCACCATCTCTAGAGACAAC GCCAAGAACACACTGTATCTGCAGATGAACTCTCTGAGAGCCGAAGACACCGCTGTG TACTACTGCGCTAGATTCTTTAGATTTAGAAACGCCATGGACTACTGGGGCCAAGGC ACACTGGTGACAGTGTCCTCC (SEQ ID NO: 19) |
| VL of mouse and chimeric C2C1A1A1 |
| DIQMTQSPASLSASVGETVTITCRTSENIYSYLAWYQQKQGKSPQFLVYNAKTLAEGVPS RFSGSGSGTQFSLNINSLQSEDFGSYYCQHYYGPPTWTFGGGTKLEIK (SEQ ID NO: 10) |
| VL of mouse C2C1A1A1 |
| GACATCCAGATGACTCAGTCTCCAGCCTCCCTATCTGCATCTGTGGGAGAAACTGTC ACCATCACATGTCGAACAAGTGAGAATATTTACAGTTATTTAGCATGGTATCAGCAGA AACAGGGAAAATCTCCTCAGTTCCTGGTCTATAATGCAAAACCTTAGCAGAAGGTGT GCCATCAAGGTTCAGTGGCAGTGGATCAGGCACACAGTTTTCTCTGAATATCAACAG CCTGCAGTCTGAAGATTTTGGGAGTTATTACTGTCAACATTATTATGGTCCTCCCACG TGGACGTTCGGTGGAGGCACCAAGCTGGAAATCAAA (SEQ ID NO: 20) |

(continued)

| Description Sequence/SEQ ID NO. |
| --- |
| VL of chimeric C2C1A1A1 |
| GACATCCAGATGACACAGAGCCCCGCCAGCCTGTCCGCCTCCGTTGGAGAGACCGT GACCATCACCTGTAGGACCTCCGAGAATATCTACAGCTACCTGGCCTGGTATCAACA GAAGCAGGGCAAGTCCCCTCAGTTTCTGGTGTACAACGCCAAGACCCTGGCCGAGG GCGTGCCCTCTAGGTTCTCCGGCTCCGGCAGCGGCACCCAGTTCAGCCTGAATATC AACAGCCTGCAGAGCGAGGACTTTGGCAGCTACTACTGTCAGCACTACTACGGCCCT CCCACCTGGACATTTGGCGGCGGCACAAAGCTGGAGATCAAG (SEQ ID NO: 21) |
| VL of huC2C1A1A1-V1, huC2C1A1A1-V5, huC2C1A1A1-V9, and huC2C1A1A1-V13 |
| DIQMTQSPSSLSASVGDRVTITCRTSENIYSYLAWYQQKPGKAPKX$_1$LX$_2$YNAKTLAEGVP SRFSGSGSGTDFTLTISSLQPEDFATYYCQHYYGPPTWTFGQGTKVEIK (SEQ ID NO: 11) X$_1$ = L, X$_2$ = I |
| DIQMTQSPSSLSASVGDRVTITCRTSENIYSYLAWYQQKPGKAPKLLIYNAKTLAEGVPSR FSGSGSGTDFTLTISSLQPEDFATYYCQHYYGPPTWTFGQGTKVEIK |
| VL of huC2C1A1A1-V2, huC2C1A1A1-V6, huC2C1A1A1-V10, and huC2C1A1A1-V14 |
| DIQMTQSPSSLSASVGQRVTITCRTSENIYSYLAWYQQKQGKPPRFLIYNAKTLAEGVPS RFSGSGSGTEFTLTITSLQAEDFGVYYCQHYYGPPTWTFGPGTKLEIK (SEQ ID NO: 12) |
| VL of huC2C1A1A1-V3, huC2C1A1A1-V7, huC2C1A1A1-V11, and huC2C1A1A1-V15 |
| DIQMTQSPSSLSASVGDRVTITCRTSENIYSYLAWYQQKPGKAPKX$_1$LX$_2$YNAKTLAEGVP SRFSGSGSGTDFTLTISSLQPEDFATYYCQHYYGPPTWTFGQGTKVEIK (SEQ ID NO: 11) X$_1$ = F, X$_2$ = V |
| DIQMTQSPSSLSASVGDRVTITCRTSENIYSYLAWYQQKPGKAPKFLVYNAKTLAEGVPS RFSGSGSGTDFTLTISSLQPEDFATYYCQHYYGPPTWTFGQGTKVEIK GACATCCAGATGACCCAGAGCCCTAGCTCTCTGAGCGCTTCCGTGGGAGATAGAGT GACCATCACATGCAGAACCTCCGAGAACATCTACAGCTATCTGGCTTGGTATCAGCA GAAGCCCGGCAAGGCCCCCAAGTTCCTGGTGTACAACGCCAAGACACTGGCTGAGG GCGTGCCTAGCAGATTCAGCGGCTCCGGCAGCGGCACAGACTTTACACTGACAATC AGCTCTCTGCAACCCGAGGACTTCGCCACCTACTACTGCCAGCACTACTATGGCCCC CCTACATGGACCTTTGGCCAAGGCACCAAGGTGGAGATCAAG (SEQ ID NO: 22) |
| VL of huC2C1A1A1-V4, huC2C1A1A1-V8, huC2C1A1A1-V12, and huC2C1A1A1-V16 |
| DIQMTQSPSSLSASVGDRVTITCRTSENIYSYLAWYQQKPGKAPKX$_1$LX$_2$YNAKTLAEGVP SRFSGSGSGTDFTLTISSLQPEDFATYYCQHYYGPPTWTFGQGTKVEIK (SEQ ID NO: 11) X$_1$ = F, X$_2$ = I |
| DIQMTQSPSSLSASVGDRVTITCRTSENIYSYLAWYQQKPGKAPKFLIYNAKTLAEGVPS RFSGSGSGTDFTLTISSLQPEDFATYYCQHYYGPPTWTFGQGTKVEIK |

(continued)

| Description<br>Sequence/SEQ ID NO. |
|---|
| Heavy chain constant region of chimeric and humanized antibodies |
| ASTKGPSVFPLAPCSRSTSESTAALGCLVKDYFPEPVTVSWNSGALTSGVHTFPAVLQS<br>SGLYSLSSVVTVPSSSLGTKTYTCNVDHKPSNTKVDKRVESKYGPPCPPCPAPEFLGGP<br>SVFLFPPKPKDTLMISRTPEVTCVVVDVSQEDPEVQFNWYVDGVEVHNAKTKPREEQFN<br>STYRVVSVLTVLHQDWLNGKEYKCKVSNKGLPSSIEKTISKAKGQPREPQVYTLPPSQEE<br>MTKNQVSLTCLVKGFYPSDIAVEWESNGQPENNYKTTPPVLDSDGSFFLYSRLTVDKSR<br>WQEGNVFSCSVMHEALHNHYTQKSLSLSLGK (SEQ ID NO: 13) |
| GCCAGCACAAAGGGCCCTTCCGTGTTTCCCCTGGCCCCCTGCAGCAGGAGCACCTC<br>TGAGTCCACCGCCGCCCTGGGCTGTCTGGTGAAGGACTACTTTCCCGAGCCCGTGA<br>CCGTGAGCTGGAATTCCGGCGCCCTGACATCCGGCGTGCACACCTTCCCCGCCGTG<br>CTGCAGTCCTCCGGCCTGTACAGCCTGAGCTCCGTGGTGACAGTGCCTTCCTCCTC<br>CCTGGGCACCAAGACCTACACATGTAATGTGGATCACAAGCCCAGCAACACAAAGGT<br>GGATAAGAGAGTGGAGTCCAAGTACGGCCCTCCTTGCCCTCCCTGTCCTGCCCCAG<br>AGTTCCTGGGCGGCCCCTCTGTGTTCCTGTTCCCCCCTAAGCCCAAGGACACACTGA<br>TGATCTCCAGGACCCCTGAGGTGACCTGCGTGGTGGTGGACGTGAGCCAGGAGGA<br>CCCTGAGGTGCAGTTCAATTGGTACGTGGATGGCGTGGAGGTGCACAATGCCAAGA<br>CAAAGCCCAGAGAGGAGCAGTTTAATTCCACATACAGGGTGGTGTCCGTGCTGACC<br>GTGCTGCACCAGGATTGGCTGAACGGCAAGGAGTACAAGTGTAAGGTGAGCAACAA<br>GGGCCTGCCTTCCTCCATCGAGAAGACAATCAGCAAGGCCAAGGGCCAGCCTAGGG<br>AGCCCCAGGTGTACACACTGCCTCCCAGCCAGGAGGAGATGACCAAGAACCAGGTG<br>AGCCTGACCTGCCTGGTGAAGGGCTTCTACCCTAGCGACATCGCCGTGGAGTGGGA |
| GTCCAACGGCCAGCCCGAGAATAACTACAAGACAACACCCCCCGTGCTGGATTCCG<br>ATGGCAGCTTCTTTCTGTACTCCAGGCTGACCGTGGATAAGAGCAGGTGGCAGGAG<br>GGCAATGTGTTCAGCTGCTCCGTGATGCACGAGGCCCTGCACAATCACTACACCCA<br>GAAGAGCCTGTCCCTGAGCCTGGGCAAGTGA (SEQ ID NO: 23) |
| Light chain constant region of chimeric and humanized antibodies |
| RTVAAPSVFIFPPSDEQLKSGTASVVCLLNNFYPREAKVQWKVDNALQSGNSQESVTEQ<br>DSKDSTYSLSSTLTLSKADYEKHKVYACEVTHQGLSSPVTKSFNRGEC* (SEQ ID NO: 14)<br>CGTACGGTGGCGGCGCCATCTGTCTTCATCTTCCCGCCATCTGATGAGCAGTTGAAA<br>TCTGGAACTGCCTCTGTTGTGTGCCTGCTGAATAACTTCTATCCCAGAGAGGCCAAA<br>GTACAGTGGAAGGTGGATAACGCCCTCCAATCGGGTAACTCCCAGGAGAGTGTCAC<br>AGAGCAGGACAGCAAGGACAGCACCTACAGCCTCAGCAGCACCCTGACGCTGAGCA<br>AAGCAGACTACGAGAAACACAAAGTCTACGCCTGCGAAGTCACCCATCAGGGCCTG<br>AGCTCGCCCGTCACAAAGAGCTTCAACAGGGGAGAGTGTTGA (SEQ ID NO: 24) |
| Human IL4Rα-his |

(continued)

| Description Sequence/SEQ ID NO. |
|---|
| MGWLCSGLLFPVSCLVLLQVASSGNMKVLQEPTCVSDYMSISTCEWKMNGPTNCSTEL RLLYQLVFLLSEAHTCIPENNGGAGCVCHLLMDDVVSADNYTLDLWAGQQLLWKGSFKP SEHVKPRAPGNLTVHTNVSDTLLLTWSNPYPPDNYLYNHLTYAVNIWSENDPADFRIYNV TYLEPSLRIAASTLKSGISYRARVRAWAQCYNTTWSEWSPSTKWHNSYREPFEQHHHH HHHHHHHH (SEQ ID NO: 15) |
| Marmoset monkey IL4Rα-his |
| MGWLCSGLLFPVSYLVLLQVAGSGSMKVLQEPTCVSDYISLSTCEWKMGGPTNCSAEL RLVYQLVFLISETNMCVPENNGAAGCVCHLFMEDMVGADNYTLDLWAGQQLLWKGSFK PSEHVKPKAPENLTVYTNVSETLLLTWSNPYPPDNYLYEKLTYAVNIWNENDPTDSRIYD VTYQEPTLRIAASTLKSGVSYRARVRAWAQSYNSTWSEWSPSTKWYNAYKEPFEKHHH HHHHHHHH (SEQ ID NO: 16) |

**Claims**

1. A pharmaceutical composition, comprising: (a) an anti-IL4Rα antibody or an antigen-binding fragment thereof, (b) a buffering agent, (c) a surfactant, and (d) a stabilizer.

2. The pharmaceutical composition according to claim 1, wherein the anti-IL4Rα antibody or the antigen-binding fragment thereof comprises a heavy chain variable region CDR1, a heavy chain variable region CDR2, a heavy chain variable region CDR3, a light chain variable region CDR1, a light chain variable region CDR2, and a light chain variable region CDR3; the heavy chain variable region CDR1, the heavy chain variable region CDR2, the heavy chain variable region CDR3, the light chain variable region CDR1, the light chain variable region CDR2, and the light chain variable region CDR3 comprise amino acid sequences having at least 85% identity to the amino acid sequences set forth in SEQ ID NOs: 1, 2, 3, 4, 5, and 6, respectively.

3. The pharmaceutical composition according to claim 1 or 2, wherein the anti-IL4Rα antibody or the antigen-binding fragment thereof comprises a heavy chain variable region comprising an amino acid sequence having at least 85% identity to the amino acid sequence set forth in SEQ ID NO: 7, 8, or 9, wherein the amino acid sequence of SEQ ID NO: 8 is:

   EVQLVESGGGLVQPGGSLRLSCAASGFTFSTYGMSWVRQAPGKGLVX$_1$VX$_2$TINSNGGSTSYPDSVK GRFTISRDNAKNTLYLQMNSLRAEDTAVYYCARFFRFRNAMDYWGQGTLVTVSS, wherein in SEQ ID NO: 8, X$_1$ = W and X$_2$ = S, X$_1$ = L and X$_2$ = A, or X$_1$ = W and X$_2$ = A; and/or
   the anti-IL4Rα antibody or the antigen-binding fragment thereof comprises a light chain variable region comprising an amino acid sequence having at least 85% identity to the amino acid sequence set forth in SEQ ID NO: 10, 11, or 12, wherein the amino acid sequence of SEQ ID NO: 11 is:
   DIQMTQSPSSLSASVGDRVTITCRTSENIYSYLAWYQQKPGKAPKX$_1$LX$_2$YNAKTLAEGVPSRFSGSG SGTDFTLTISSLQPEDFATYYCQHYYGPPTWTFGQGTKVEIK, wherein in SEQ ID NO: 11, X$_1$ = L and X$_2$ = I, X$_1$ = F and X$_2$ = V, or X$_1$ = F and X$_2$ = I.

4. The pharmaceutical composition according to any one of claims 1-3, wherein the anti-IL4Rα antibody or the antigen-binding fragment thereof comprises a heavy chain variable region and a light chain variable region; the heavy chain variable region and the light chain variable region comprise: (1) amino acid sequences having at least 85% identity to the amino acid sequences set forth in SEQ ID NOs: 7 and 10, respectively; (2) amino acid sequences having at least 85% identity to the amino acid sequences set forth in SEQ ID NOs: 8 and 11, respectively, wherein in SEQ ID NO: 8, X$_1$ = W and X$_2$ = S; (3) amino acid sequences having at least 85% identity to the amino acid sequences set forth in SEQ ID NOs: 8 and 12, respectively, wherein in SEQ ID NO: 8, $X_1$ = W and $X_2$ = S; (4) amino acid sequences having at least 85% identity to the amino acid sequences set forth in SEQ ID NOs: 9 and 11, respectively; (5) amino acid sequences having at least 85% identity to the amino acid sequences set forth in SEQ ID NOs: 9 and 12, respectively; (6) amino acid sequences having at least 85% identity to the amino acid sequences set forth in SEQ ID NOs: 8 and 11, respectively, wherein in SEQ ID NO: 8, $X_1$ = L and $X_2$ = A; (7) amino acid sequences having at least 85% identity to the amino acid sequences set forth in SEQ ID NOs: 8 and 12, respectively, wherein in SEQ ID NO: 8, $X_1$ = L and $X_2$ = A; (8) amino acid sequences having at least 85% identity to the amino acid sequences set forth in SEQ ID NOs: 8 and 11, respectively, wherein in SEQ ID NO: 8, $X_1$ = W and $X_2$ = A; or (9) amino acid sequences having at least 85% identity to the amino acid sequences set forth in SEQ ID NOs: 8 and 12, respectively, wherein in SEQ ID NO: 8, $X_1$ = W and $X_2$ = A; wherein the amino acid sequence of SEQ ID NO: 8 is:

EVQLVESGGGLVQPGGSLRLSCAASGFTFSTYGMSWVRQAPGKGLV$X_1$V$X_2$TINSNGGSTSYPDSVK GRFTISRDNAKNTLYLQMNSLRAEDTAVYYCARFFRFRNAMDYWGQGTLVTVSS;

wherein the amino acid sequence of SEQ ID NO: 11 is: DIQMTQSPSSLSASVGDRVTITCRTSENIYSYLAWYQQKPGKAPK$X_1$L$X_2$YNAKTLAEGVPSRFSGSG SGTDFTLTISSLQPEDFATYYCQHYYGPPTWTFGQGTKVEIK, wherein in SEQ ID NO: 11, $X_1$ = L and $X_2$ = I, $X_1$ = F and $X_2$ = V, or $X_1$ = F and $X_2$ = I.

5. The pharmaceutical composition according to any one of claims 1-4, wherein the anti-IL4Rα antibody or the antigen-binding fragment thereof further comprises a heavy chain constant region comprising the amino acid sequence set forth in SEQ ID NO: 13, and a light chain constant region comprising the amino acid sequence set forth in SEQ ID NO: 14.

6. The pharmaceutical composition according to any one of claims 1-5, wherein the concentration of the anti-IL4Rα antibody or the antigen-binding fragment thereof is 30 mg/mL to 300 mg/mL, 50 mg/mL to 250 mg/mL, 70 mg/mL to 200 mg/mL, 100 mg/mL to 180 mg/mL, 120 mg/mL to 180 mg/mL, 120 mg/mL to 150 mg/mL, or 150 mg/mL to 180 mg/mL.

7. The pharmaceutical composition according to any one of claims 1-6, wherein the buffering agent includes a phosphate buffering agent, an acetate buffering agent, or a histidine salt buffering agent; preferably, the phosphate buffering agent is a sodium phosphate buffering agent, the acetate buffering agent is a sodium acetate-acetic acid buffering agent, and/or the histidine salt buffering agent is a histidine-histidine hydrochloride buffering agent;
optionally, the concentration of the buffering agent is 1 mM to 100 mM, 2 mM to 80 mM, 4 mM to 60 mM, 8 mM to 40 mM, 10 mM to 30 mM, 10 mM to 20 mM, or 20 mM to 30 mM.

8. The pharmaceutical composition according to any one of claims 1-7, wherein the surfactant includes polysorbate 80 or polysorbate 20;
optionally, the concentration of the surfactant is 0.01 mg/mL to 2 mg/mL, 0.05 mg/mL to 1 mg/mL, 0.1 mg/mL to 0.8 mg/mL, 0.2 mg/mL to 0.6 mg/mL, or 0.2 mg/mL to 0.4 mg/mL.

9. The pharmaceutical composition according to any one of claims 1-8, wherein the stabilizer is selected from one or more of trehalose, mannitol, sucrose, arginine or a pharmaceutically acceptable salt thereof, proline, and sodium chloride;
optionally, the concentration of sucrose is 10 mg/mL to 100 mg/mL, 20 mg/mL to 80 mg/mL, 30 mg/mL to 60 mg/mL, 40 mg/mL to 50 mg/mL, or 50 mg/mL to 60 mg/mL; the concentration of arginine or the pharmaceutically acceptable salt thereof is 10 mM to 100 mM, 20 mM to 80 mM, 30 mM to 60 mM, 40 mM to 50 mM, or 50 mM to 60 mM; the concentration of proline is 60 mM to 600 mM, 80 mM to 500 mM, 100 mM to 450 mM, 120 mM to 400 mM, 150 mM to 350 mM, 200 mM to 350 mM, or 250 mM to 350 mM; and/or the concentration of sodium chloride is 1 mM to 80 mM, 2 mM to 60 mM, 4 mM to 50 mM, 8 mM to 40 mM, 10 mM to 30 mM, or 10 mM to 20 mM.

10. The pharmaceutical composition according to any one of claims 1-9, wherein the pH of the pharmaceutical composition is 4 to 7, 5 to 7, 5 to 6.5, 5.3 to 6.5, 5.3 to 6.3, 5.3 to 5.8, or 5.8 to 6.3.

11. The pharmaceutical composition according to any one of claims 1-10, wherein the pharmaceutical composition comprises:

(a) 30 mg/mL to 300 mg/mL of the anti-IL4Rα antibody or the antigen-binding fragment thereof,
(b) 1 mM to 100 mM of the buffering agent,
(c) 0.01 mg/mL to 2 mg/mL of the surfactant, and
(d) the stabilizer, including trehalose, mannitol, sucrose, arginine or a pharmaceutically acceptable salt thereof, proline, and/or sodium chloride,
wherein the pH of the pharmaceutical composition is 4 to 7, 5 to 7, 5 to 6.5, 5.3 to 6.5, 5.3 to 6.3, 5.3 to 5.8, or 5.8 to 6.3;
optionally, the pharmaceutical composition comprises:

(a) 30 mg/mL to 300 mg/mL of the anti-IL4Rα antibody or the antigen-binding fragment thereof,
(b) 1 mM to 100 mM of the phosphate buffering agent, the acetate buffering agent, or the histidine salt buffering agent,
(c) 0.01 mg/mL to 2 mg/mL of polysorbate 80 or polysorbate 20, and
(d) 10 mg/mL to 100 mg/mL of sucrose, 10 mM to 100 mM of arginine or the pharmaceutically acceptable salt thereof, 60 mM to 600 mM of proline, and/or 1 mM to 80 mM of sodium chloride,

wherein the pH of the pharmaceutical composition is 4 to 7, 5 to 7, 5 to 6.5, 5.3 to 6.5, 5.3 to 6.3, 5.3 to 5.8, or 5.8 to 6.3.

12. A lyophilized formulation comprising an anti-IL4Rα antibody or an antigen-binding fragment thereof, wherein the lyophilized formulation prepared by lyophilizing the pharmaceutical composition according to any one of claims 1-11; or the lyophilized formulation is capable of forming the pharmaceutical composition according to any one of claims 1-11 upon reconstitution.

13. A method for treating an allergic disease associated with excessive IL4 and/or IL13 signaling in a subject, comprising: administering to the subject the pharmaceutical composition according to any one of claims 1-11 or the lyophilized formulation according to claim 12; preferably, the allergic disease is atopic dermatitis, anaphylaxis, allergic rhinitis, or allergic asthma.

14. A method for treating a tumor associated with increased STAT6 activation in a subject, comprising:

administering to the subject the pharmaceutical composition according to any one of claims 1-11 or the lyophilized formulation according to claim 12; preferably, the tumor is a solid tumor; more preferably, the tumor is melanoma, lung cancer, kidney cancer, prostate cancer, cervical cancer, colorectal cancer, gastric cancer, pancreatic cancer, ovarian cancer, and/or urothelial cancer.

15. A method for reducing IL4 and/or IL13 signaling or reducing type 2 immune response in a subject, comprising: administering to the subject the pharmaceutical composition according to any one of claims 1-11 or the lyophilized formulation according to claim 12; preferably, the IL4 and/or IL13 signaling is manifested by the activation and/or proliferation of B cells, eosinophils and/or macrophages, the proliferation of fibroblasts, and the proliferation of smooth muscle; preferably, the proliferation of smooth muscle cells is the proliferation of airway smooth muscle cells.

<div align="center">**INTERNATIONAL SEARCH REPORT**</div>

| | International application No. |
|---|---|
| | **PCT/CN2022/114652** |

**A. CLASSIFICATION OF SUBJECT MATTER**

A61K 39/395(2006.01)i; C07K 16/24(2006.01)i; A61K 38/17(2006.01)i; C07K 16/28(2006.01)i; A61P 35/00(2006.01)i; A61P 37/00(2006.01)i

According to International Patent Classification (IPC) or to both national classification and IPC

**B. FIELDS SEARCHED**

Minimum documentation searched (classification system followed by classification symbols)

A61K, C07K, A61P

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VEN, CJFD, CNKI, PUBMED, ELSVIER, 万方, WANFANG, 百度学术搜索, BAIDU SCHOLAR SEARCH, Genbank, 中国专利生物序列检索系统, Chinese Patent Biological Sequence Retrieval System: 单抗, 肿瘤, 缓冲剂, 表面活性剂, 稳定剂, C2C1A1A1, IL-4Rα, IL-4R, monoclonal antibody, mAb, tumor, cancer, buffer, surfactant, stabilizer, 对SEQ ID NOs: 1-14的序列检索, Sequence Search for SEQ ID NOs: 1-14

**C. DOCUMENTS CONSIDERED TO BE RELEVANT**

| Category* | Citation of document, with indication, where appropriate, of the relevant passages | Relevant to claim No. |
|---|---|---|
| PX | WO 2021170020 A1 (CHIA TAI TIANQING PHARMACEUTICAL GROUP CO., LTD.) 02 September 2021 (2021-09-02) claims 1-20 | 1-15 |
| X | CN 108409860 A (SHANGHAI MABGEEK BIOTECHNOLOGY CO., LTD.) 17 August 2018 (2018-08-17) see claims 9 and 10, and description, paragraph [0045] | 1, 6-13, 15 |
| Y | CN 108409860 A (SHANGHAI MABGEEK BIOTECHNOLOGY CO., LTD.) 17 August 2018 (2018-08-17) see claims 9 and 10, and description, paragraph [0045] | 5 |
| X | CN 101600456 A (WYETH LLC.) 09 December 2009 (2009-12-09) see claims 15, 16, and 21, and description, p. 74, paragraph 4, p. 76, paragraph 1, and p. 77, the last paragraph | 1, 6-15 |
| Y | CN 101600456 A (WYETH LLC.) 09 December 2009 (2009-12-09) see claims 15, 16, and 21, and description, p. 74, paragraph 4, p. 76, paragraph 1, and p. 77, the last paragraph | 5 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 November 2022** | **30 November 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/114652**

**C.    DOCUMENTS CONSIDERED TO BE RELEVANT**

| Category* | Citation of document, with indication, where appropriate, of the relevant passages | Relevant to claim No. |
|---|---|---|
| Y | CN 111961651 A (BEIJING JIADEHE CELL THERAPY TECHNOLOGY CO., LTD.) 20 November 2020 (2020-11-20) see claim 3, and sequence table, SEQ ID NO: 16 | 5 |
| A | WO 2010053751 A1 (REGENERON PHARMA et al.) 14 May 2010 (2010-05-14) see abstract | 1-15 |

Form PCT/ISA/210 (second sheet) (January 2015)

**INTERNATIONAL SEARCH REPORT**

International application No.

**PCT/CN2022/114652**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

         ☑ in the form of an Annex C/ST.25 text file.

         ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

         ☐ in the form of an Annex C/ST.25 text file (Rule 13*ter*.1(a)).

         ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    [1] The actually submitted sequence table is an XML file in Standard ST.26.

Form PCT/ISA/210 (continuation of first sheet) (January 2015)

**INTERNATIONAL SEARCH REPORT**

International application No.

**PCT/CN2022/114652**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **13-15**
   because they relate to subject matter not required to be searched by this Authority, namely:

   [1] Claims 13-15 relate to a method for treating diseases or a method for reducing signal transduction or immune responses in a subject, both comprising the step of administering a drug to the subject, being both treatment methods for treating a human body, and belonging to the subject matter as defined in PCT Rule 39.1(iv) that does not warrant a search conducted by the international searching authority. A search was conducted on the basis that claims 13-15 are reasonably expected as pharmaceutical use claims, i.e., the subject matter thereof is reasonably expected as "a use of the pharmaceutical composition according to any one of claims 1-11 or the lyophilized preparation according to claim 12 in the preparation of a drug for treating allergic diseases associated with excess IL4 and/or IL13 signal transduction in a subject", "a use of the pharmaceutical composition according to any one of claims 1-11 or the lyophilized preparation according to claim 12 in the preparation of a drug for treating tumors associated with increased STAT6 activation in a subject", and "a use of the pharmaceutical composition according to any one of claims 1-11 or the lyophilized preparation according to claim 12 in the preparation of a preparation for reducing IL4 and/or IL13 signal transduction in a subject or reducing type 2 immune responses in a subject".

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

| International application No. |
| --- |
| **PCT/CN2022/114652** |

| Patent document cited in search report | | | Publication date (day/month/year) | Patent family member(s) | | | Publication date (day/month/year) |
| --- | --- | --- | --- | --- | --- | --- | --- |
| WO | 2021170020 | A1 | 02 September 2021 | BR | 112022017174 | A2 | 18 October 2022 |
| | | | | AU | 2021228061 | A1 | 13 October 2022 |
| | | | | TW | 202140564 | A | 01 November 2021 |
| CN | 108409860 | A | 17 August 2018 | | None | | |
| CN | 101600456 | A | 09 December 2009 | | None | | |
| CN | 111961651 | A | 20 November 2020 | | None | | |
| WO | 2010053751 | A1 | 14 May 2010 | NI | 201100064 | A | 02 November 2011 |
| | | | | US | 2014271681 | A1 | 18 September 2014 |
| | | | | HR | P20130467 | T1 | 30 June 2013 |
| | | | | CY | 1114123 | T1 | 27 July 2016 |
| | | | | PL | 2356151 | T3 | 30 August 2013 |
| | | | | SV | 2011003880 | A | 15 August 2011 |
| | | | | HU | E039212 | T2 | 28 December 2018 |
| | | | | ES | 2802241 | T3 | 18 January 2021 |
| | | | | DK | 2356151 | T3 | 17 June 2013 |
| | | | | EP | 3351560 | A1 | 25 July 2018 |
| | | | | JP | 2019055963 | A | 11 April 2019 |
| | | | | AU | 2009311496 | A1 | 14 May 2010 |
| | | | | RU | 2011120194 | A | 10 December 2012 |
| | | | | PH | 12014500462 | A1 | 01 June 2015 |
| | | | | HK | 1159136 | A1 | 27 July 2012 |
| | | | | TW | 201919700 | A | 01 June 2019 |
| | | | | IL | 211726 | D0 | 30 June 2011 |
| | | | | LT | PA2018002 | I1 | 12 February 2018 |
| | | | | LT | 3064511 | T | 10 July 2018 |
| | | | | EP | 3715372 | A1 | 30 September 2020 |
| | | | | NL | 300922 | I2 | 15 January 2019 |
| | | | | PT | 3064511 | T | 17 July 2018 |
| | | | | JP | 2021087440 | A | 10 June 2021 |
| | | | | US | 2013078675 | A1 | 28 March 2013 |
| | | | | JP | 2022160404 | A | 19 October 2022 |
| | | | | US | 2018179288 | A1 | 28 June 2018 |
| | | | | HU | S1800014 | I1 | 28 March 2018 |
| | | | | SI | 2356151 | T1 | 31 July 2013 |
| | | | | RS | 52782 | B | 31 October 2013 |
| | | | | PL | 3351560 | T3 | 21 September 2020 |
| | | | | EP | 2356151 | A1 | 17 August 2011 |
| | | | | JP | 2016041069 | A | 31 March 2016 |
| | | | | CO | 6362024 | A2 | 20 January 2012 |
| | | | | IN | 2884CHENP2011 | A | 31 August 2012 |
| | | | | UA | 102122 | C2 | 10 June 2013 |
| | | | | JP | 2012507294 | A | 29 March 2012 |
| | | | | RU | 2014147773 | A | 20 June 2016 |
| | | | | NZ | 591922 | A | 30 March 2012 |
| | | | | CR | 20110148 | A | 27 April 2011 |
| | | | | PE | 20100738 | A1 | 03 November 2010 |
| | | | | PT | 3351560 | T | 11 May 2020 |
| | | | | MA | 32802 | B1 | 01 November 2011 |
| | | | | CA | 2737044 | A1 | 14 May 2010 |
| | | | | AR | 073978 | A1 | 15 December 2010 |

Form PCT/ISA/210 (patent family annex) (January 2015)

INTERNATIONAL SEARCH REPORT
Information on patent family members

| Patent document cited in search report | Publication date (day/month/year) | Patent family member(s) | | Publication date (day/month/year) |
|---|---|---|---|---|
| | | RS | 57522 B1 | 31 October 2018 |
| | | DK | 3064511 T3 | 16 July 2018 |
| | | PL | 3064511 T3 | 28 September 2018 |
| | | HN | 2011001210 A | 22 October 2013 |
| | | NZ | 596093 A | 30 November 2012 |

International application No.

**PCT/CN2022/114652**

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2021077784 W

**Non-patent literature cited in the description**

- **STEPHEN J. GALLI et al.** *Nature,* 2008, vol. 454 (7203), 445-454
- **GRUNING G et al.** *Science,* 1998, vol. 282, 2261-2263
- **RANKIN JA et al.** *Proc Natl Acad Sci USA,* 1996, vol. 93, 7821-7825
- **WILLS-KARP M et al.** *Science,* 1998, vol. 282, 2258-2261
- **IRINA G. LUZINA et al.** *J Leukoc Biol,* 2012, vol. 92 (4), 753-764
- **HELLER NM et al.** *Sci Signal,* 2008, vol. 1 (51), ra17-ra17
- **GADANI SP et al.** *J Immunol,* 2012, vol. 189, 4213-4219
- **NAPPO G et al.** *Oncogenesis,* 2017, vol. 6 (5), e342
- Peptide and Protein Drug Delivery. Marcel Dekker, Inc, 1991, 247-301
- **JONES, A.** *Adv. Drug Delivery Rev.,* 1993, vol. 10, 29-90
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **BRUMMELL et al.** *Biochem,* 1993, vol. 32, 1180-8
- **DE WILDT et al.** *Prot. Eng.,* 1997, vol. 10, 835-41
- **KOMISSAROV et al.** *J. Biol. Chem.,* 1997, vol. 272, 26864-26870
- **HALL et al.** *J. Immunol.,* 1992, vol. 149, 1605-12
- **KELLEY ; O'CONNELL.** *Biochem.,* 1993, vol. 32, 6862-35
- **ADIB-CONQUY et al.** *Int. Immunol.,* 1998, vol. 10, 341-6
- **BEERS et al.** *Clin. Can. Res.,* 2000, vol. 6, 2835-43